# EUROPÄISCHE PATENTSCHRIFT

(11) **EP 2 150 619 B1**
(45) Veröffentlichungstag und Bekanntmachung des Hinweises auf die Patenterteilung: **22.07.2015**
(21) Anmeldenummer: 08757965.2
(22) Anmeldetag: 16.04.2008
(51) Int. Cl.: C12P 7/46, C12N 1/19, C12N 9/04, C12N 9/88

(54) **MIKROORGANISMUS ZUR HERSTELLUNG VON BERNSTEINSÄURE**
MICROORGANISM FOR THE PRODUCTION OF SUCCINIC ACID
MICROORGANISME DESTINÉ À LA PRODUCTION D'ACIDE SUCCINIQUE

(30) Priorität: 20.04.2007 DE 102007019184
(43) Veröffentlichungstag der Anmeldung: 10.02.2010
(73) Patentinhaber: Organo Balance GmbH, 13355 Berlin (DE)
(72) Erfinder: LANG, Christine, 10625 Berlin (DE); VEEN, Markus, 84549 Engelsburg (DE); BÖTTNER, Mewes, 10407 Berlin (DE); RAAB, Andreas, 13156 Berlin (DE)
(74) Vertreter: Jungblut, Bernhard Jakob
(86) Internationale Anmeldenummer: PCT/DE2008/000670
(87) Internationale Veröffentlichungsnummer: WO 2008/128522

(56) Entgegenhaltungen:
- EP-A- 1 672 077
- WO-A-2006/034156
- MINARD K I ET AL: "GLUCOSE-INDUCED DEGRADATION OF THE MDH2 ISOZYME OF MALATE DEHYDROGENASE IN YEAST" JOURNAL OF BIOLOGICAL CHEMISTRY, Bd. 267, Nr. 24, 1992, Seiten 17458-17464, XP002493350 ISSN: 0021-9258 in der Anmeldung erwähnt
- SANCHEZ A M ET AL: "Efficient succinic acid production from glucose through overexpression of pyruvate carboxylase in an Escherichia coli alcohol dehydrogenase and lactate dehydrogenase mutant" BIOTECHNOLOGY PROGRESS, XX, XX, Bd. 21, Nr. 2, 2. März 2005 (2005-03-02), Seiten 358-365, XP002391565 ISSN: 8756-7938
- BLOM JOLANDA ET AL: "Redirection of the respiro-fermentative flux distribution in Saccharomyces cerevisiae by overexpression of the transcription factor Hap4p" APPLIED AND ENVIRONMENTAL MICROBIOLOGY, Bd. 66, Nr. 5, Mai 2000 (2000-05), Seiten 1970-1973, XP002493372 ISSN: 0099-2240
- ARIKAWA Y ET AL: "Effect of gene disruptions of the TCA cycle on production of succinic acid in Saccharomyces cerevisiae" JOURNAL OF BIOSCIENCE AND BIOENGINEERING, ELSEVIER, AMSTERDAM, NL, Bd. 87, Nr. 1, 1. Januar 1999 (1999-01-01), Seiten 28-36, XP003017715 ISSN: 1389-1723
- LOPEZ-ERRASQUIN ET AL: "Real-Time RT-PCR assay to quantify the expression of fum1 and fum19 genes from the Fumonisin-producing Fusarium verticillioides", JOURNAL OF MICROBIOLOGICAL METHODS, ELSEVIER, AMSTERDAM, NL, vol. 68, no. 2, 27 January 2007 (2007-01-27), pages 312-317, XP005863292, ISSN: 0167-7012, DOI: 10.1016/J.MIMET.2006.09.007

## Beschreibung

### Gebiet der Erfindung

Die Erfindung betrifft einen Mikroorganismus mit einer trunkierten Varianten des Gens MDH₂, einem heterologen Fumarase-Gen, einem überexprimiertem Gen FRDS1, und einem überexprimierten Gen PYC1 und/oder PYC 2, Verwendungen eines solchen Mikroorganismus und ein Verfahren zu dessen Herstellung.

### Stand der Technik und Hintergrund der Erfindung

Dicarbonsäuren haben ein großes ökonomisches Potential, weil sie als Vorläufersubstanzen für zahlreiche Chemikalien verwendet werden können. Zum Beispiel dient Bernsteinsäure als Vorstufe für die Herstellung von Kunststoffen auf der Basis von 1,4-Butanediol, Tetrahydrofuran und Gamma-Butyrolacton. Bernsteinsäure wird heute chemisch durch katalytische Hydrierung von Maleinsäureanhydrid zu Bersteinsäureanhydrid und nachfolgender wasseranlagerung, bzw. durch direkte katalytische Hydrierung von Maleinsäure hergestellt.

Bernsteinsäure wird auch von vielen Mikroorganismen ausgehend von Zuckern oder Aminosäuren unter physiologischen Umgebungsbedingungen gebildet. Unter anaeroben Bedingungen werden in der Regel neben Bernsteinsäure weitere Gärendprodukte wie Ethanol, Milchsäure, Essigsäure und Ameisensäure gebildet. Die Biosynthese von Bernsteinsäure mit ihrem hohen Sauerstoffanteil erfordert eine reduktive CO₂-Fixierung. Bernsteinsäure ist ein Metabolit, der normalerweise durch anaerobe Fermentationsprozesse angereichert wird. Während die Ausbeute und Anreicherung des Produktes unter anaeroben Bedingungen vielfach besser ist als unter aeroben Bedingungen, liegt der Nachteil in einem ausschließlich anaeroben Prozess in einer technischen Begrenzung der Biomassen-Herstellung und einer geringen Produktivität des mikrobiellen Produzenten. Somit ist ein relativ geringer Biomassen/Produkt Wirkungsgrad die Folge. Zudem ist es schwierig, strikt anaerobe Mikroorganismen technisch zu handhaben.

Verschiedene Mikroorganismen, die in Lage sind unter anaeroben Bedingungen Bernsteinsäure zu synthetisieren, sind bekannt. Die Literaturstelle US 5,143,834 beschreibt eine Variante von A. *succiniciproducens.* Es handelt sich dabei um einen obligat anaeroben Mikroorganismus, der nur moderate Mengen an Bernsteinsäure herstellen kann und zudem nicht in der Lage ist, hohe osmotische Drücke und Salzkonzentrationen zu tolerieren. Die Literaturstelle US 7,063,968 beschreibt ein mikrobielles Isolat aus Rinder-Pansen, *Mannheimia* sp. 55E, welches in der Lage ist, sowohl unter aeroben als auch anaeroben Bedingungen organische Säuren zu synthetisieren. Dabei handelt es sich allerdings nicht um eine spezifische Anreicherung von Bernsteinsäure, sondern um ein Gemisch aus verschiedenen organischen Säuren, wie Ameisensäure, Essigsäure, Milchsäure und Bernsteinsäure. Nachteil dieses Produzenten ist, dass eine ökonomische Verwendung des Stammes nur schwer möglich, wenn nicht unmöglich ist, da zur Gewinnung von Bernsteinsäure aufwendige Anreicherungs- und Aufreinigungsverfahren angewendet werden müssten. Die Literaturstelle US 5,869,301 beschreibt ein Verfahren zur Herstellung von Dicarbonsäuren in einem zweistufigen Fermentationsprozesses mit *E*. *coli* AFP-111, wobei in einer ersten Phase mikrobielle Biomasse unter aeroben Bedingungen hergestellt und in einer zweiten Phase die Produktion von Bernsteinsäure anaerob durchgeführt wird. Die erste Phase der Biomassengewinnung ist in diesem Prozess limitiert, da die Glukose-Konzentration im Fed-Batch Prozess auf 1 g/L beschränkt sein muss, um eine Acetat-Anreicherung zu vermeiden, die sowohl den Biomassengewinnungsprozess, als auch die Bernsteinsäureproduktion stört. Somit ist die Biomassengewinnung mit diesem Prozess nur begrenzt möglich. Des weiteren unterliegt der Biosyntheseweg für die Bernsteinsäure in diesem Prozess einer starken Katabolit-Repression, da Gene der Glykolyse, des Zitronensäurezyklus und Glyoxylatweges durch Glukose stark reprimiert sind, wie aus der Literaturstelle DeRisi J.L., Iyer V.R., Brown P.O., Science. 278(5338): 680 - 686 (1997) bekannt. Das hat zur Folge, dass die Synthese von Bernsteinsäure in Gegenwart von Glukose weitestgehend reprimiert und damit stark begrenzt ist.

Aus der Literaturstelle US 6,190,914 sind Mikroorganismen bekannt, bei welchen durch Modulation von geeigneten Transkriptionsfaktoren und Kinasen die Glucose-Repression diverser Gene vermindert wird. Die Herstellung von organischen Säuren mittels solcher Mikroorganismen, insbesondere aber auch weitergehend für die Produktion organischer Säuren optimierte Mikroorganismen, sind nicht entnehmbar.

Genetische Modifikationen an Mikroorganismen zur Herstellung von Bernsteinsäure sind aus den Literaturstellen EP 1672077 A und ARIKAWA Y et al: "Effect of gene disruptions of the TCA cycle on production of succinic acid in Saccharomyces cerevisiae", JOURNAL OF BIOSCIENCE AND BIOENGINEERING, Bd. 87, Nr. 1, 1. Januar 1999, Seiten 28-36, bekannt.

### Technisches Problem der Erfindung

Der Erfindung liegt daher das technische Problem zu Grunde, ein Verfahren und einen Mikroorganismus zu dessen Durchführung anzugeben, welches es ermöglicht, Biomasse ohne Limitierungen im Fermentationsprozess zu gewinnen, eine Carbonsäure des Glyoxylatzyklus, insbesondere Bernsteinsäure, ohne Katabolit-Repression im Biosyntheseweg im Produktionsprozess herzustellen und eine möglichst geringe Nebenprodukt-Anreicherung während der Produktionsphase zu gewährleisten.

### Grundzüge der Erfindung und bevorzugte Ausführungsformen

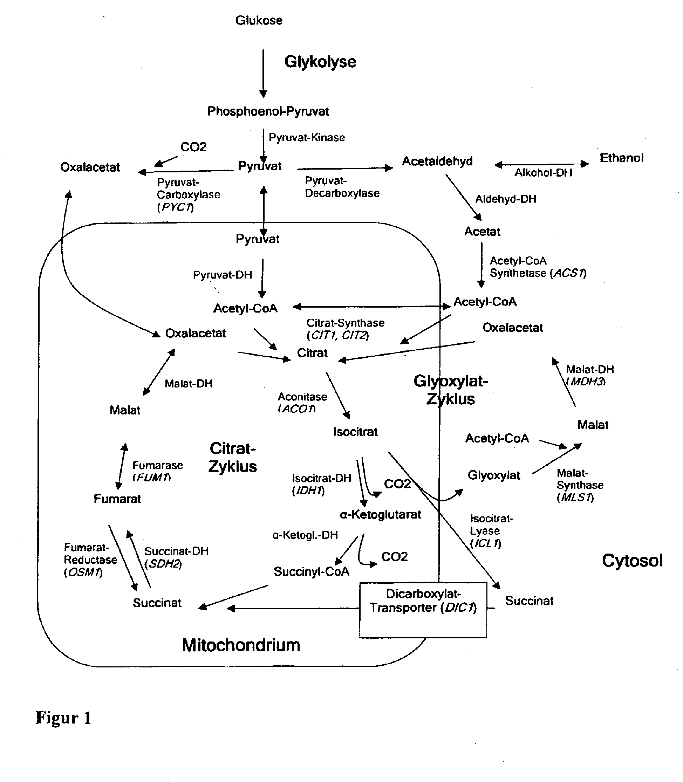

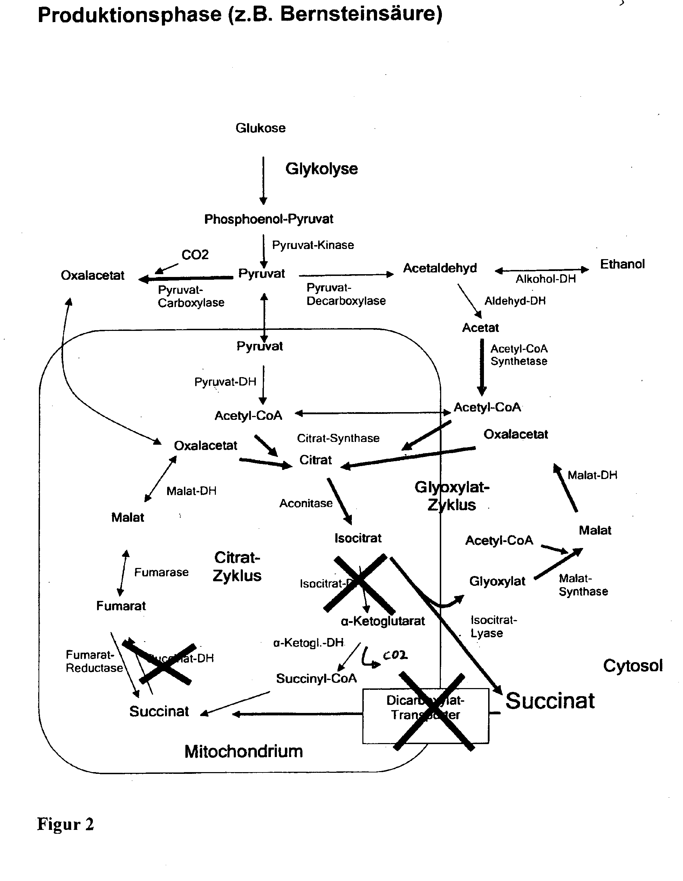

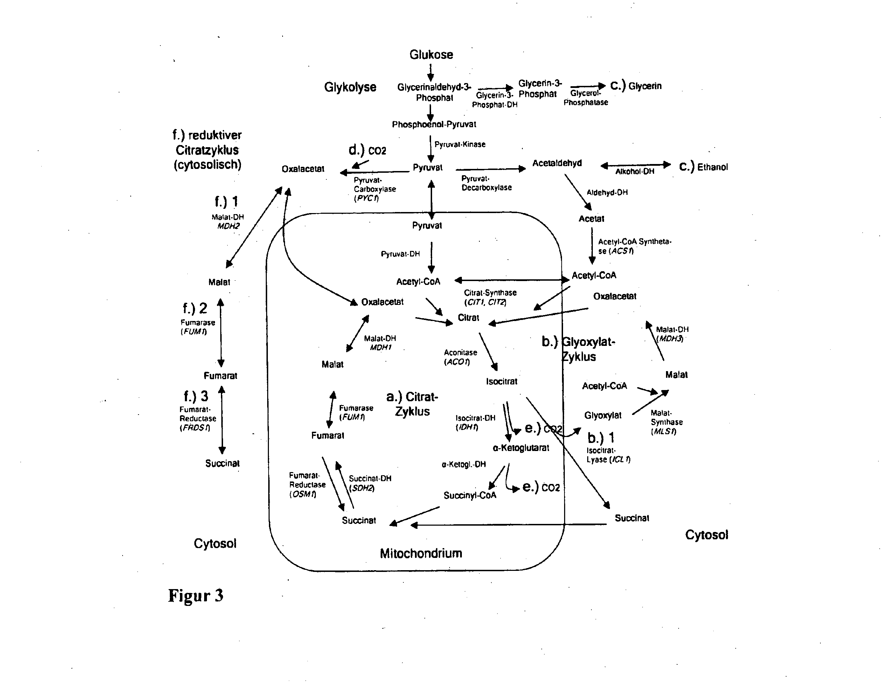

Zur Lösung dieses technischen Problems lehrt die Erfindung den Gegenstand des Anspruchs 1. Die cytosolische Malat-Dehydrogenase (Mdh2p) unterliegt einem durch Glucose-induzierten verstärkten proteolytischen Abbau (Hung et al. 2004). Die cytosolische Malat-Dehydrogenase wird zur Succinat-Synthese über den reduktiven Teil des CitratZyklus, ausgehend von Pyruvat über Oxalacetat, Malat und Fumarat zu Succinat, genutzt (Figur 3 f.) 1). Der reduktive Citratzyklus, also der nicht cyclische rückwärtigen Umlauf des Citratzyklus (Figur 3 f.) ist Voraussetzung für die stöchiometrisch maximale molare Ausbeute bei der Produktion von Bernsteinsäure auf Glucose. Nur der reduktive Citratzyklus in Kombination mit dem Glyoxylatzyklus ermöglicht bei der Produktion von Bernsteinsäure maximale Ausbeuten von bis zu 1,71 mol Succinat pro mol Glucose durch Reoxidation von NADH zu NAD+ und CO₂ Fixierung.

Eine Möglichkeit die cytosolische Malat-Dehydrogenase (Mdh2p) der Glucose-induzierten proteolytischen Degradation zu entziehen, ist die Überexpression eines um die ersten 12 N-terminalen Aminosäuren, sowie, optional, des C-terminalen Prolin verkürzten Mdh2p Proteins. Die ersten 12 N-terminalen Aminosäuren, sowie das C-terminale Prolin sind essentiell für die Degradation von Mdh2p (Hung, Brown et al. 2004).

Ein weiterer Engpass bei der Succinat-Produktion über den reduktiven Teil des Citratzyklus ist die Reaktion von Malat zu Fumarat (Figur 3 f.) 2). Katalysiert wird diese Umsetzung durch das Enzym Fumarase (Fumlp). Es ist eines der wenigen Enzyme des respirativen zentralmetabolismus, welches keine Isoenzyme aufweist. Trotzdem ist es sowohl im Cytosol, als auch im Mitochondrium lokalisiert. Die Lokalisation des singulären Translationsproduktes wird über die N-terminale mitochondriale Targeting-Sequenz und die Proteinfaltung bestimmt (Sass et al. 2003).

Die Umsetzung von Malat zu Fumarat ist thermodynamisch sehr ungünstig, da die Fumarase eine 17-fach höhere Affinität zu Fumarat, als zu Malat aufweist. Deshalb ist eine effiziente Nutzung des reduktiven Teils des Citratzyklus zur Produktion von Succinat nicht mit der nativen Fumarase der Hefe Saccharomyces cerevisiae möglich.

Um diese Problematik zu überwinden, muss ein Fumarase-Enzym heterolog in der Hefe exprimiert werden, welche vorzugsweise aus einem Organismus stammt, welcher natürlicherweise eine gemischte Säuregärung betreibt. In diesen Organismen läuft der reduktive Citratzyklus viel effizienter, da bei der gemischten Säuregärung Succinat ein natürliches Gärendprodukt darstellt. Die Thermodymamik der Fumarase solcher Organismen ist im Bezug auf die Umsetzung von Malat zu Fumarat viel günstiger, als in Saccharomyces cerevisiae. Als Spenderorganismen für eine heterolog exprimierte Fumarase kommen zum Beispiel *Escherichia coli, Anaerobiospirillum succiniciproducens, Actinobacillus succinogenes, Mannheimia succiniciproducens, Corynebacterium glutamicum* in Frage.

Der letzte Schritt zur Succinat-Produktion über den reduktiven Citratzyklus ist die Umsetzung von Fumarat zu Succinat. Diese Reaktion wird im Cytosol durch das Enzym Fumarat-Reduktase (Frdslp, Figur 3 f.) 3). katalysiert. Eine Überexpression dieses Enzyms steigert die Effizienz der reduktiven Succinat-Produktion.

Die trunkierte Variante des Gens MDH2 codiert vorzugsweise für ein MDH2 Protein, welches um das C-terminale Prolin verkürzt ist.

Die Transformation von Hefezellen kann in fachüblicher Weise erfolgen und hierzu wird auf die Literaturstellen Schiestl R.H., et al., Curr Genet. Dec, 16(5-6):339-346 (1989), oder Manivasakam P., et al., Nucleic Acids Res. Sep 11, 21(18):4414-4415 (1993), oder Morgan A.J., Experientia Suppl., 46:155-166 (1983) verwiesen.

Zur Transformation geeignete Vehikel, insbesondere Plasmide, sind beispielsweise aus den Literaturstelle Naumovski L., et al., J Bacteriol, 152(1) : 323-331 (1982), Broach J.R., eta al., Gene, 8(1):121-133 (1979), Sikorski R.S., et al., Genetics, 122(1)_19-27 (1989). Bei diesen vectoren handelt es sich um Yep24, Yep13, pRS-Vektorserie, sowie um YCp19 oder pYEXBX.

Die Herstellung von im Rahmen der Erfindung geeigneten Expressionskassetten erfolgt typischerweise durch Fusion des Promotors mit der für das Gen kodierenden Nukleinsäuresequenz und ggf. einem Terminator nach üblichen Rekombinations- und Klonierungstechniken, wie beispielsweise in den Literaturstellen Maniatis T., et al., Molecular Cloning: A Laboratiry Manual, Cold Spring Harbor Laboratory, Cold Spring Harbor, NY, USA, 1989, oder Sihlavy T.J., et al., Experiments with Gene Fusions, Cold Spring Harbor Laboratory, Cold Spring Harbor, NY, USA, 1984, oder Ausubel F.M., et al., Current Protocols in Molecular Biology, Greene Publishing Assoc. and Wiley-Interscience, 1987, beschrieben.

Der Mikroorganismus kann eine Hefe, insbesondere ausgewählt aus der Gruppe bestehend aus "Saccharomyces cerevisiae, Saccharomyces, Saccharomycecopsis, Saccharomycodes, Schizosaccharomyces, Wickerhamia, Debayomyces, Hansenula, Hanseniaspora, Pichia, Kloeckera, Candida, Zygosaccharomyces, Ogataea, Kuraishia, Komagataella, Yarrowia, Metschnikowia, Williopsis, Nakazawaea, Kluyveromyces, Cryptococcus, Torulaspora, Bullera, Rhodotorula, Willopsis, Kloeckera und Sporobolomyces", sein, und das Gen FUM1 kann aus einem Organismus aus der Gruppe bestehend aus "Escherichia coli, Anaerobiospirillum, Actinobacillus, Mannheimia, Corynebacterium" ausgewählt sein.

Zumindest eines der Gene aus der Gruppe bestehend aus "FRDS1, PYC1 und PYC2" kann unter der Kontrolle eines zweiten fremden, konstitutiv aktiven Promotors, beispielsweise des ADH1-Promotors, stehen.

Vorzugsweise sind beide natürlichen Gene MDH2 und FUM1 ersetzt oder ergänzt und/oder wobei beide Gene FRDS1 und PYC1 überexprimiert.

Ein solcher Mikroorganismus ist zur Herstellung von Bernsteinsäure mit beachtlich erhöhter Ausbeute einsetzbar. Dies erfolgt dadurch, dass a) der Mikroorganismus optional in einem Wachstumskulturmedium unter aeroben Bedingungen kultiviert und vermehrt wird, b) anschließend der Mikroorganismus unter optional anaeroben Bedingungen kultiviert wird und c) anschließend an Stufe b) oder während der Stufe b) die Bernsteinsäure aus dem Kulturüberstand abgetrennt und optional aufgereinigt wird.

Im Rahmen des erfindungsgemäßen Verfahrens ist es bevorzugt, wenn die Stufe a) bis zu einer Zelldichte von zumindest 100 g Biotrockenmasse/l, vorzugsweise zumindest 120 g/l, höchstvorzugsweise zumindest 140 g/l, durchgeführt wird. Die Stufe b) kann bis zu einer Carbonsäurekonzentration von zumindest 0,4 Mol/l, vorzugsweise zumindest 0,8 Mol/l, höchstvorzugsweise zumindest 1,0 Mol/1, durchgeführt werden. In Stufe a) kann ein pH im Bereich von 4 bis 9, vorzugsweise von 6 bis 8, und eine Salzkonzentration im Bereich von 0,01 bis 0,5 Mol/1, vorzugsweise von 0,05 bis 0,2 Mol/l, höchstvorzugsweise von 0,05 bis 0,1 Mol/l, eingestellt werden. In Stufe b) kann ein pH im Bereich von 4 bis 9, vorzugsweise von 6 bis 8, und eine Salzkonzentration im Bereich von 0,01 bis 0,5 Mol/l, vorzugsweise von 0,05 bis 0,2 Mol/l, höchstvorzugsweise von 0,05 bis 0,1 Mol/l, eingestellt werden. Die Stufe a) wird vorzugsweise bei einer Temperatur von 20 bis 35 °C, vorzugsweise von 28 bis 30 °C, und für eine Dauer von 1 bis 1000 h, vorzugsweise von 2 bis 500 h, höchstvorzugsweise 2 bis 200 h, durchgeführt. In Stufe b) ist eine Durchführung bei einer Temperatur von 15 bis 40 °C, vorzugsweise von 20 bis 35 °C, höchstvorzugsweise 28 bis 30 °C, und für eine Dauer von 1 bis 1000 h, vorzugsweise von 2 bis 500 h, höchstvorzugsweise 2 bis 200 h, bevorzugt.

Als Kulturmedien für die Stufe a) kommt beispielsweise WMVIII-Medium (Lang C., Looman A.C., Appl Microbiol Biotechnol. 44(1-2) : 147-156 (1995)) in Frage. Dabei liegt die Tetracyclin-Menge im Kulturmedium vorzugsweise unterhalb von 20 mg/l, bevorzugt unterhalb von 10 mg/l. höchstvorzugsweise, unterhalb von 1 mg/l, bis hin zu Werten, die unterhalb der Nachweisgrenze liegen.

Als Kulturmedium für die Stufe b) kommt beispielsweise WMVIII-Medium in Frage, auch ist ein übliches Melasse-Medium einsetzbar. Dabei liegt die Tetracyclin-Menge bevorzugt oberhalb von 1 mg/l, höchstvorzugsweise oberhalb von 3 mg/l. Als Bereiche kommen beispielsweise 1 bis 3 mg/l oder 3 bis 15 mg/l in Frage.

Die Stufe c) kann anschließend an die Stufe b) durchgeführt werden. Dann wird der Kulturüberstand von den Mikroorganismen abgetrennt, beispielsweise durch Filtration oder Zentrifugation. Die Stufe c) kann aber auch während der Stufe b) durchgeführt werden, und zwar kontinuierlich oder diskontinuierlich. In letzterem Falle wird zumindest ein Teil des Kulturüberstandes entnommen und durch neues Kulturmedium ersetzt und dieser Vorgang ggf. mehrfach wiederholt. Aus dem entnommenen Kulturüberstand wird die Bernsteinsäure gewonnen. Eine kontinuierliche Abtrennung kann über geeignete Membranen öder durch Leiten eines Stromes des Kulturmediums über eine Vorrichtung zur Abtrennung der Bernsteinsäure erfolgen.

Ein erfindungsgemäßer Mikroorganismus ist herstellbar, indem in dem wild-Typ Mikroorganismus oder in einem gentechnisch gegenüber dem wild-Typ veränderten oder mutierten Ausgangsorganismus einer der wild-Typ Gene MDH2 oder FUM1, oder beide dieser Gene, deletiert oder inaktiviert oder ergänzt wird, indem die respektive Variante des MDH2 Gens und/oder das fremde FUM1 Gen in den Mikroorganismus eingeführt und der Mikroorganismus hiermit transformiert wird und/oder indem zumindest eines der Gene FRDS1, PYC1 und PYC2, vorzugsweise zumindest beide Gene FRDS1 und PYC1, unter der Kontrolle des zweiten Promotors in den Mikroorganismus eingeführt und der Mikroorganismus hiermit transformiert wird.

Ein weiterer Vorteil des respirativen Modus für die Produktion von Dicarbonsäuren ist die verstärkte Expression des Enzyms Pyruvat-Carboxylase (Pyclp und Pyc2p), welches die Umsetzung von Pyruvat zu Oxalacetat katalysiert, wobei Kohlenstoff in Form von CO₂ fixiert wird (siehe Figur 3 d)). Die enzymatische Umsetzung der Pyruvat-Carboxylase stellt den ersten Schritt des reduktiven Teils des Citratzyklus (Figur 3 f.)), sowie der Gluconeogenese dar. Die Gluconeogenese, also der Aufbau des Speicherstoffs Glycogen, findet vor allem unter Atmungsbedingungen statt (Haurie, Perrot et al. 2001).

Die CO₂ Fixierung bei diesem enzymatischen Schritt ermöglicht einen Prozess mit ausgeglichener oder sogar positiver CO₂ Bilanz, was auf prozesstechnischer Seite eine große Umweltrelevanz darstellt.

Des Weiteren wird in einer folgend erläuterten Weiterbildung der Erfindung durch die Propagierung der Atmung die Nebenproduktbildung in Form von Glycerin und Ethanol (siehe Figur 3 c.)) und somit Ausbeuteverluste vermindert, da Glycerin und Ethanol die Gärendprodukte der Hefe darstellen.

Die Veränderungen des Expressionsprofils im Verlauf des "diauxischen shifts" betrifft knapp ein Viertel der etwa 6000 Gene in *Saccharomyces cerevisiae* (Young et al. 2003). Diese tiefgreifenden Veränderungen werden durch einige wenige Kinasen und Transkriptionsfaktören der Katabolit-Repressionskaskade induziert, welche jeweils im Promotorbereich einer Vielzahl von Zielgenen regulatorische Wirkung entfalten. Dies macht deutlich, daß neben den Genen des respirativen Zentralmetabolismus eine enorme Anzahl weiterer Gene durch Glucose reprimiert ist. Zur effizienten Produktion von organischen Säuren in Hefe reicht es deshalb nicht aus ausschließlich die Gene des Zentralmetabolismus durch Überexpression transkriptionell zu deregulieren. Es ist nötig eine Vielzahl weiterer Glucose-reprimierter Gene des respirativen Systems dieser Repression zu entziehen. Dies betrifft Gene, welche für mitochondriale Transportsysteme, Austausch von Redoxequivalenten, Proteine der Atmungskette und die mitochondriale Biogenese kodieren. In einem Hefestamm, welcher keine oder eine verminderte Glucose-Repression aufweist, läuft das gesamte respirative System auf primären und anderen Kohlenstoffquellen effizienter. Da Dicarbonsäuren, wie Bernsteinsäure, Fumarsäure, Apfelsäure und Oxalessigsäure Intermediate des respirativen Metabolismus sind, ist ein solcher Stamm für die Produktion dieser Säuren sehr geeignet. Da aus Gründen der Stammstabilität und der Effizienz nur eine begrenzte Zahl an Genen einzeln transkriptionell dereguliert werden kann, kann die Aufhebung der Glucose induzierten Repression einer Vielzahl von Genen nur durch Beeinflussung von geeigneten Transkriptionsfaktoren, welche für diese Repression verantwortlich sind, auf übergeordneter Ebene erreicht werden.

In der Hefe *Saccharomyces cerevisiae* sind mehrere Kinasen und Transkriptionsfaktoren der Katabolit-Repressionskaskade bekannt und untersucht, welche im Promotorbereich ihrer Zielgene des respirativen Systems repressiv wirken. Des Weiteren sind Transkriptionsfaktoren bekannt und untersucht, welche im Verlauf des "diauxischen shifts" zu einer verstärkten Expression ihrer Zielgene führen, also induktiv bzw. aktivierend wirken. Zur Aufhebung oder Verminderung der Glucose-Repression auf übergeordneter Ebene, und somit zur Aktivierung vieler Gene des respirativen Systems, müssen geeignete Kinasen und Transkriptionsfaktoren reprimiert oder aktiviert werden. So kann die Effizienz der Produktion von organischen Säuren des respirativen Systems gesteigert werden.

Zusammenfassend bietet ein Hefestamm mit verminderter oder aufgehobener Glucose-Repression, durch Aktivierung oder Repression geeigneter Kinasen und Transkriptionsfaktoren, bei der Herstellung von organischen Dicarbonsäuren des respirativen Zentralmetabolismus auf primären Kohlenstoffquellen im Vergleich zu einem Wildtypstamm folgende Vorteile:
▪ der Kohlenstofffluss durch den Citrat- und Glyoxylatzyklus ist erhöht
▪ der Stamm weist ein höheres und schnelleres Wachstum auf
▪ weniger Nebenproduktbildung in Form von Ethanol und Glycerin
▪ der Glyoxylatzyklus wird propagiert
▪ die Enzyme, welche für die CO₂ Fixierung im Zentralmetabolismus verantwortlich sind, werden verstärkt exprimiert
▪ sämtliche Gene des respirativen Systems, z.B. der mitochondrialen Transpörtsysteme, der Atmungskette, der mitochondrialen Biogenese und für den Austausch von Redoxequivalenten sind aktiv
▪ Enzyme des Citrat- und Glyoxylatzyklus werden der Glucose-induzierten negativen Regulation auf Proteinebene entzogen

Diese Vorteile führen zu einer effizienteren Produktion hinsichtlich der Produktions -dauer und -ausbeuten. Außerdem wird die Ökoeffizienz gesteigert, indem weniger CO₂ gebildet und mehr CO₂ fixiert wird.

Die Hefezelle weist nicht nur auf Glucose die Katabolit-Repression auf, sondern auch auf anderen fermentierbaren C₆- und C₅- Zuckern, wie zum Beispiel Galactose oder Ribose, und in geringerem Ausmaß auch auf nicht fermentierbaren Kohlenstoffquellen, wie zum Beispiel Glycerin (Gancedo 1998). Deshalb ist eine effizientere Produktion in einem Glucose dereprimierten Stamm auf sämtlichen fermentierbaren, sowie auf vielen nicht fermentierbaren Kohlenstoffquellen zu erwarten.

In diesem Zusammenhang sind einerseits Transkriptionsfaktoren zu nennen.

Der Snflp/Snf4p Komplex gehört zu den Protein Serin/Threonin Kinasen und spielt bei der Aufhebung der Glucose-Repression im Zuge des "diauxischen shifts" eine zentrale Rolle. Der Snfl/Snf4 Komplex ist in der Hirarchie der Kaskade, welche für die Glucose-Repression bzw. Derepression verantwortlich ist, sehr weit oben anzusiedeln. Im Verlauf des "diauxischen shifts" wird der der Snf1p/Snf4p durch die Snf1-Kinasen Sak1p, Tos1p und Elmlp phosphoryliert und somit aktiviert (Rubenstein et al. 2006). Der aktivierte Komplex ist im Kern lokalisiert, wo er zwei in der Hirarchieebene weiter unten anzusiedelnde Transkripitionsfaktoren beeinflusst. Dies sind die Proteine Miglp und Cat8p (Treitel et al. 1998).

Das Zink- Finger-Protein Miglp ist ein transkriptioneller Repressor, welcher die Proteine Tuplp und Cyc8p rekrutiert, um dann als Komplex an die entsprechende Consensus-Sequenz des Promotors einer Vielzahl von Glucose-reprimierten Genen zu binden, wodurch die Transkription des nachgeschalteten Gens verhindert wird (Treitel and Carlson 1995).

Cat8p ist ein transkriptioneller Aktivator und induziert die Expression von mindestens 34 Genen, wenn keine Glucose mehr im Medium vorhanden ist. Dies sind vor allem die Gene des Glyoxylatzyklus, welcher für eine effiziente Produktion von organischen Säuren in Hefe benötigt wird. Des Weiteren unterliegen auch einige Gene, welche für den intrazellulären Transport von Intermediaten des Glyoxylat-und Citratzyklus verantwortlich sind der Regulation durch Cat8p (Haurie, Perrot et al. 2001).

Der aktive Snflp/Snf4p Komplex phosphoryliert das Mig1 Protein, wodurch dieses inaktiviert und aus dem Zellkern translokalisiert wird (De Vit et al. 1997) und phosphoryliert Cat8p und Siplp, dessen funktionelles Homolog, was zu einer Aktivierung dieser beiden Proteine führt.

Somit trägt ein im Zellkern lokalisierter, aktiver Snflp/Snf4p -Komplex wesentlich zur Aufhebung der Glucose-Repression bei, da er weitere Transkriptionsfaktoren der Repressionskaskade unmittelbar beeinflusst. Dieser Komplex setzt sich aus den Proteinen Snflp und Snf4p, sowie jeweils einem der Proteine Gal83p, Siplp und Sip2p zusammen (Jiang and Carlson 1997). Die letztgenannten Proteine haben Einfluss auf die Lokalisation des Gesamtkomplexes innerhalb der Zelle und stellen das Bindeglied des Komplexes dar. Snf4p ist die regulatorische und Snflp die katalytische Untereinheit. Snflp wird aktiviert, indem eine der 3 redundanden Kinasen Saklp, Toslp oder Elmlp die Aminosäure Threonin an Position 210 phosphoryliert. Dieser Phosphorylierung wirkt die Glc7p/Reglp Typ1 Protein-Phosphatase entgegen (Santangelo 2006). Reglp ist die regulatorische Untereinheit dieser Protein-Phosphatase und leitet diesen Komplex durch Bindung an Glc7p zu einer Reihe von Substraten, die in verschiedenen Prozessen einschließlich der Glucose-Repression, des Zellwachstums, und der Glykogen-Bildung involviert sind (Cui et al. 2004). Reglp interagiert mit der Kinase-Domäne des Snf1 Proteins und leitet Glc7p zur Aktivierungsschleife von Snflp, was in der Dephosphorylierung und Inaktivierung von Snflp, und somit in der Glucose-Repression vieler Gene resultiert.

Frederick et al. (1996) konnten ein Homolog zu *Reg1p* identifizieren- *Reg2p.* Eine *Δreg1-* oder *Δreg2*-Mutante zeigt ein deutlich verlangsamtes Wachstum auf fermentierbaren und nicht-fermentierbaren Kohlenstoffquellen. Eine *Δreg1Δreg2*-Doppelmutante zeigt noch gravierendere Mängel (Frederick and Tatchell 1996). Die Überexpression von *REG2* in einer *Δreg1Δreg2*-Mutante mildert diese Mängel im Bezug auf das Wachstum, die stark verminderte Glucose-Repression bleibt jedoch unbeeinflusst. Dies zeigt, daß vor allem Reglp eine Rolle bei der Aufrechterhaltung der Glucose-Repression spielt.

Um Snflp/Snf4p auch in Anwesenheit von Glucose in einem aktiven Zustand zu halten, bieten sich 2 Mechanismen an. Zum einem ist das Protein Saklp, welches als Hauptkinase für die Phosphorylierung und somit die Aktivierung des Snflp/Snf4p Komplexes verantwortlich ist, durch transkriptionelle Deregulation des Gens *SAK1* über zu exprimieren.

Des Weiteren ist die Dephosphorylierung durch die Glc7p/Reglp Protein-Phosphatase zu verhindern. Die Repression bzw. Deletion von *GLC7* führt zu einer nicht lebensfähigen Zelle, weshalb dies nur durch Repression bzw. Deletion des Gens *REG1* realisiert werden kann.

Durch beide Maßnahmen kann das regulatorische Duell zwischen Saklp und Glc7p/Reglp in Richtung eines aktiven Snflp/Snf4p-Konplexes verschoben werden, was wesentlich zur Aufhebung der Glucose-Repression beiträgt.

Der Hap2/3/4/5 Proteinkomplex reguliert eine große Zahl von Glucose-reprimierten Genen. Dies sind vor allem Gene der Atmungskette, sowie des Citratzyklus. Als Aktivator induziert er die Transkription dieser Gene während des "diauxischen shift" und trägt stark zur Verschiebung des respiro-fermentativen Gleichgewichts in Richtung Respiration bei. Die Gene *HAP2, HAP3* und *HAP5* werden konstitutiv exprimiert (McNabb and Pinto 2005). *HAP4* wird natürlicherweise nur bei Wachstum auf nicht fermentierbaren Kohlenstoffquellen exprimiert. Bei der Kultivierung einer *HAP4*-Überexpressionsmutante wurde beobachtet, daß diese Mutation im Vergleich zum Wildtyp zu einer erhöhten Wachstumsrate, Biomasse- und Acetat-Produktion, sowie zu einer reduzierten Ethanol und Glycerin Produktion führt (Blom et al. 2000). Der Hap2/3/4/5 Proteinkomplex propagiert das respirative System indem der Kohlenstofffluss durch den Citratzyklus und die respiratorische Kapazität erhöht, sowie die mitochondriale Biogenese gefördert wird.

Durch die Überexpression von Hap4p wird also die Effizienz der Produktion organischer Carbonsäuren, insbesondere Dicarbonsäuren und Hydroxyfettsäuren des respirativen Zentralmetabolismus der Hefe, wie zum Beispiel Bernsteinsäure, Fumarsäure, Apfelsäure, Oxalessigsäure hinsichtlich der Produktionsdauer und -ausbeuten gesteigert.

Die meisten Gene, welcher der Glucose-Repression unterliegen, sind durch mehrere Transkriptionsfaktoren reguliert. Im Promotorbereich dieser Gene sind sehr häufig Bindungsdomänen für mehrere Transkriptionsfaktoren, wie zum Beispiel Miglp, Hap2/4/5/6p, Cat8p usw. zu finden. Auch regulieren sich diese Proteine gegenseitig. Die Transkription von Cat8p zum Beispiel unterliegt der strikten Repression in Anwesenheit von Glucose, vor allem durch das Migl Protein, welches auch die Transkriptions des Gens *SNF1* reprimiert (Schuller and Entian 1991). Im Promotorbereich des Gens *HAP4* wurde ebenfalls eine Miglp Bindungssequenz identifiziert (Gancedo 1998). Es konnte gezeigt werden, daß die Beeinflussung mehrerer Transkriptionsfaktoren der Glucose-Repressionkaskade einen additiven Effekt zeigt (Lascaris et al. 2004). Es wird also deutlich, daß erst die kombinierte Beeinflussung von mehr als einem Transkriptionsfaktor- bzw. Komplex zu einer umfassenden Reduzierung bzw. Ausschaltung der Katabolit-Repression führen kann.

Daher ist auch ein isolierter gentechnisch veränderter Mikroorganismus beschrieben als Weiterbildung der vorstehend beschriebenen Erfindung, wobei eines der Gene HAP4 oder SAK1, überexprimiert wird, oder wobei beide Gene HAP4 und SAK1 überexprimiert werden, und/oder wobei das Gen REG1 reprimiert oder deletiert ist.

Dabei handelt es sich vorzugsweise um eine Crabtreepositive Hefe, insbesondere ausgewählt aus der Gruppe bestehend aus "Saccharomyces cerevisiae, Saccharomyces, Saccharomycecopsis, Saccharomycodes, Schizosaccharomyces, Wickerhamia, Debayomyces, Hansenula, Hanseniaspora, Pichia, Kloeckera, Candida, zygosaccharomyces, Ogataea, Kuraishia, Komagatäella, Yarrowia, Metschnikowia, Williopsis, Nakazawaea, Kluyveromyces, Cryptococcus, Torulaspora, Bullera, Rhodotorula, Willopsis, Kloeckera und Sporobolomyces".

Im Einzelnen kann eines der Gene HAP4 oder SAK1, oder können beide Gene, unter der Kontrolle eines zweiten fremden, konstitutiv aktiven Promotors, insbesondere des ADH1-Promotors, stehen.

Solche Mikroorganismen sind generell zur Herstellung organischer Carbonsäuren, insbesondere von Bernsteinsäure, Fumarsäure, Apfelsäure, Oxalessigsäure, Milchsäure, Essigsäure, und/oder Ameisensäure, mit erhöhten Ausbeuten geeignet. Welche Carbonsäure mit besonders hoher Ausbeute gebildet wird, hängt von den weiteren gentechnischen Maßnahmen, wie vorstehend beschrieben, ab. Bezüglich der Herstellung der Carbonsäuren gelten die vorstehend angebrachten Ausführungen analog.

Schließlich betrifft die Weiterbildung ein Verfahren zur Herstellung eines solchen Mikroorganismus, wobei in dem wild-Typ Mikroorganismus oder in einem gentechnisch gegenüber dem wild-Typ veränderten oder mutierten Ausgangsorganismus das wild-Typ Gen REG1 deletiert oder inaktiviert wird, und/oder wobei zumindest eines der Gene HAP4 oder SAK1, vorzugsweise beide Gene, unter der Kontrolle des zweiten Promotors in den Mikroorganismus eingeführt und der Mikroorganismus hiermit transformiert wird. Optional können zusätzlich die Maßnahmen angebracht sein, welche vorstehend im Zusammenhang mit weiteren Varianten erfindungsgemäßer Mikroorganismen beschrieben sind.

Die in verschiedenen Ausführungsformen verwendeten Gene bzw. deren Nukleinsäuresequenzen sind in der NCBI Datenbank mit den folgenden Accession No. beschrieben:
- IDH1:: Z71313 Y13139
- SDH2:: Z73146 Y13138
- DIC1:: EF059331
- FUM1:: NC001148
- OSM1:: NC001142
- PYC1:: Z72584 Y13135
- ACS1:: AY723758
- CIT1:: Z71616 Y13139
- ACO1:: M33131
- ICL1:: X65554
- MLS1:: X64407 S50520
- MDH3:: M98763
- CIT2:: Z11113
tetO und tTA: Gari E. et al., YEAST 13:837-848 (1997)
ADH1: Lang C., Looman A.C., Appl Microbiol Biotechnol. 44(1-2):147-156 (1995)
- *HAP4:*: NP_012813
- *REG1:*: NP_010311
- *SAK1:*: NP_011055
- *MDH2:*: NP_014515
- *FRDS1:*: NP_010867
- *fumB:*: NC_004431
- *aceA:*: U00096 AE000111-AE000510

Vorstehend genannte Literaturstellen weise die folgenden bibliographischen Daten auf:
Blom, J., M. J. De Mattos and L. A. Grivell (2000). "Redirection of the respiro-fermentative flux distribution in Saccharomyces cerevisiae by overexpression of the transcription factor Hap4p." Appl Environ Microbiol 66(5): 1970-3.
Cui, D. Y., C. R. Brown and H. L. Chiang (2004). "The type 1 phosphatase Reglp-Glc7p is required for the glucose-induced degradation of fructose-1,6-bisphosphatase in the vacuole." J Biol Chem 279(11): 9713-24.
De Vit, M. J., J. A. Waddle and M. Johnston (1997). "Regulated nuclear translocation of the Migl glucose repressor." Mol Biol Cell 8(8): 1603-18.
Frederick, D. L. and K. Tatchell (1996). "The REG2 gene of Saccharomyces cerevisiae encodes a type 1 protein phosphatase-binding protein that functions with Reglp and the Snfl protein kinase to regulate growth." Mol Cell Biol 16 (6) : 2922-31.
Gancedo, J. M. (1998). "Yeast carbon catabolite repression." Microbiol Mol Biol Rev 62(2): 334-61.
Haurie, V., M. Perrot, T. Mini, P. Jeno, F. Sagliocco and H. Boucherie (2001). "The transcriptional activator Cat8p provides a major contribution to the reprogramming of carbon metabolism during the diauxic shift in Saccharomyces cerevisiae." J Biol Chem 276(1): 76-85.
Hung, G. C., C. R. Brown, A. B. Wolfe, J. Liu and H. L. Chiang (2004). "Degradation of the gluconeogenic enzymes fructose-1,6-bisphosphatase and malate dehydrogenase is mediated by distinct proteolytic pathways and signaling events." J Biol Chem 279(47): 49138-50.
Jiang, R. and M. Carlson (1997). "The Snfl protein kinase and its activating subunit, Snf4, interact with distinct domains of the Sip1/Sip2/Gal83 component in the kinase complex." Mol Cell Biol 17(4): 2099-106.
Lascaris, R., J. Piwowarski, H. van der Spek, J. Teixeira de Mattos, L. Grivell and J. Blom (2004). "Overexpression of HAP4 in glucose-derepressed yeast cells reveals respiratory control of glucose-regulated genes." Microbiology 150(Pt 4): 929-34.
Lopez-Boado, Y. S., P. Herrero, T. Fernandez, R. Fernandez and F. Moreno (1988). "Glucose-stimulated phosphorylation of yeast isocitrate lyase in vivo." J Gen Microbiol 134(9): 2499-505.
McNabb, D. S. and I. Pinto (2005). "Assembly of the Hap2p/Hap3p/Hap4p/Hap5p-DNA complex in Saccharomyces cerevisiae." Eukaryot Cell 4(11): 1829-39.
Ordiz, I., P. Herrero, R. Rodicio and F. Moreno (1996). "Glucose-induced inactivation of isocitrate lyase in Saccharomyces cerevisiae is mediated by the cAMP-dependent protein kinase catalytic subunits Tpkl and Tpk2." FEBS Lett 385 (1-2) : 43-6.
Rubenstein, E. M., R. R. McCartney and M. C. Schmidt (2006). "Regulatory domains of Snfl-activating kinases determine pathway specificity." Eukaryot Cell 5(4): 620-7.
Santangelo, G. M. (2006). "Glucose signaling in Saccharomyces cerevisiae." Microbiol Mol Biol Rev 70(1): 253-82.
Sass, E., S. Karniely and O. Pines (2003). "Folding of fumarase during mitochondrial import determines its dual targeting in yeast." J Biol Chem 278(46): 45109-16.
Schuller, H. J. and K. D. Entian (1991). "Extragenic suppressors of yeast glucose derepression mutants leading to constitutive synthesis of several glucose-repressible enzymes." J Bacteriol 173(6): 2045-52.
Treitel, M. A. and M. Carlson (1995). "Repression by SSN6-TUP1 is directed by MIG1, a repressor/activator protein." Proc Natl Acad Sci U S A 92(8): 3132-6.
Treitel, M. A., S. Kuchin and M. Carlson (1998). "Snfl protein kinase regulates phosphorylation of the Migl repressor in Saccharomyces cerevisiae." Mol Cell Biol 18(11): 6273-80.
Young, E. T., K. M. Dombek, C. Tachibana and T. Ideker (2003). "Multiple pathways are co-regulated by the protein kinase Snfl and the transcription factors Adrl and Cat8." J Biol Chem 278(28): 26146-58.

Im Folgenden wird die Erfindung anhand von Ausführungsbeispielen näher erläutert. Die Beispiele 1 bis 8 sind Vergleichsbeispiele.

### Beispiel 1: Herstellung eines Mikroorganismus mit Tetracyclin-reguliertem Promotorsystem

Um eine Unterbrechung des Zitrat-Zyklus nach Beendigung des Biomassezuwachses zu erreichen, werden erfindungsgemäß drei Gene dieses Stoffwechselweges durch regulierbare Promotoren kontrolliert. Es handelt sich um das Gen *IDH1,* welches die erste Untereinheit der NAD-abhängigen Isozitrat-Dehydrogenase codiert und um das Gen *SDH2,* welches für eine Untereinheit der Succinat-Dehydrogenase codiert, sowie das Gen DIC1, welches für den mitochondrialen Dicarboxylat-Transporter kodiert. Eine Ausschaltung dieser Enzymaktivitäten hat zur Folge, dass Isozitrat, das Substrat des Glyoxylatzyklus, nicht mehr über den Zitrat-Zyklus in 2-Oxoglutarat umgewandelt wird und Bernsteinsäure nicht in Fumarat verstoffwechselt wird, sowie das Succinat aus dem Glyoxylatzyklus nicht mehr in das Mitochondrium transportiert und somit nicht mehr dem im Mitochondrium lokalisierten Zitratzyklus zugeführt wird. Der Zitrat-Zyklus ist somit unterbrochen. Die zu verwendenden. Promotoren haben die wichtige Eigenschaft, während der Wachstumsphase aktiv zu sein, um ein Funktionieren des Zitrat-Zyklus zu ermöglichen und in der Produktionsphase durch Zugabe eines Repressors möglichst stark reprimiert zu sein. Diese Regulation wird, neben dem reprimierbaren tetO-Promotor, durch die zweite Komponente des Tetracyclin-regulierten Promotorsystems ermöglicht. Hierbei handelt es sich um den transkriptionellen Transaktivator (tTA), welcher ein Fusionsprotein aus einer Operatorbindungsdomäne und einer Aktivierungsdomäne darstellt. tTA bindet an den tetO-Promotor, was die Transkription des nachgeschalteten Gens zur Folge hat. Antibiotika aus der Gruppe der Tetracycline induzieren eine Konformationsänderung des tTA, was zu dessen Ablösung vom tetO-Promotor führt und dadurch die Transkription des nachgeschalteten Gens unterbunden wird. Um die Funktion des tetO-Promotors zu gewährleisten, ist also auch das Gen für den tTA in das Genom des entsprechenden Mikroorganismus zu integrieren. Dafür steht beispielsweise der LEU2-Locus zur Verfügung.

Isocitrat-Dehydrogenase, Succinat-Dehydrogenase und Dicarboxylat-Transporter werden während der Wachstumsphase unter der Kontrolle des durch Tetracyclin reprimierbaren tetO Promotors exprimiert. Während der Produktionsphase werden die Enzymaktivitäten reguliert ausgeschaltet. *Saccharomyces cerevisiae* wird so verändert, dass die natürlichen Promotoren der drei Zielgene durch den reprimierbarenbaren Promotor z.B. durch zielgerichtete Integration in das Hefegenom ausgetauscht werden.

Die Konstruktion des so erhaltenen Hefestammes *S*. *cerevisiae* GRFura3 tTA *tetO*prom-*SDH2* und *tetO*prom*-IDH1* und *tetO*prom-*DIC1* ergibt sich aus der folgenden Tabelle 1.

**Tabelle 1**

| **Stamm** | **Beschreibung** |
|---|---|
| GRFura3 | *leu2, ura3, his3* |
| GRFura3tTA | *tTA:: Leu2, leu2, ura3, his3* |
| GRFura3 tTArepr.SDH2 | tetOprom::sdh2prom, *leu2, his3, ura3 tTA:: Leu2* |
| GRFura3 | *tTA::Leu2,tetO*prom::*sdh*2prom, *tet*Oprom::*idh1*prom, |
| repr.SDH2 | *tet*Oprom::*dic1*prom, *leu2, his3, ura3* |
| repr.IDH1 | |
| repr.DIC1 | |
| =GRFura3tTArepr.SDH2 | |
| repr.IDH1repr.DIC1 | |

### Beispiel 2: Mikroorganismus mit konstitutiv aktiver Bernsteinsäuresynthese

Da der Glyoxylatzyklus und andere Reaktionen auf dem Weg zur Bernsteinsäurebiosynthese natürlicherweise in der Hefezelle in Anwesenheit von Glukose transkriptiönell stark reprimiert sind, mit der Folge, dass dieser Biosyntheseweg nicht aktiv ist, werden alle, oder eine Auswahl von Enzymen dieses Stoffwechselweges und die Pyruvat-Carboxylase, dem Enzym, welches für die anaplerotischen Auffüllreaktion (CO₂ Fixierung) verantwortlich ist, während der Produktionsphase zur Biosynthese von Bernsteinsäure aus Glukose transkriptionell vorzugsweise dereguliert. Für die deregulierte Expression der Gene in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glukose und Ethanol unabhängigen, Expression führt (siehe Lang C., Looman A.C., Appl Microbiol Biotechnol. 44(1-2): 147-156. (1995)). Transkriptionell dereguliert werden die Gene PYC1, ACS1, CIT1, ACO1, ICL1, MLS1, MDH3 und CIT2. Zu diesem Zweck wird ein Expressionssystem verwendet, welches die gleichzeitige transkripionell deregulierte Expression von PYC1, ACS1, CIT1, ACO1, ICL1, MLS1, MDH3 und CIT2 ermöglicht. Die einzelnen Gene (PYC1, ACS1, CIT1, ACO1, ICL1, MLS1, MDH3 und CIT2) wurden aus chromosomaler Hefe-DNA des Stammes *S. cerevisiae* S288c mittels PCR amplifiziert und mit Restriktionslinkern versehen und anschließend einzeln in das Hefe Chromosom unter der Kontrolle des konstitutiven *ADH1* Promotors integriert.

Die Konstruktion des so erhaltenen Hefestammes *S*. *cerevisiae* ergibt sich aus der Tabelle 2.

**Tabelle 2**

| **Stamm** | **Beschreibung** |
|---|---|
| GRFura3tTa | *tTA::Leu2, tet*Oprom*::sdh*2prom, *tetO*₇prom*::idh1*prom, |
| repr.SDH2 | *tetO*prom*::dic1*prom *his3, ura3, leu2* |
| repr.IDH1 | |
| repr.DIC1 | |
| GRFura3tTA | *tTA::Leu2, tetOprom::sdh2prom, tetO₇*prom*::idh1*prom, |
| repr.SDH2 | *tetO₇*prom*::dic1*prom, *der.PYC1::pyc1, his3, ura3, leu2* |
| repr.IDH1 | |
| repr.DIC1 | |
| der. PYC1 | |
| GRFura3tTA | *tTA::Leu2, tetO*prom*::sdh2*prom, *tetOprom::idh1*prom, |
| repr.SDH2 | *tetO*prom*::dic1*prom, *der.PYC1::pyc1, der.ACS1::acs1, his3, ura3,* |
| repr.IDH1 | *leu2* |
| repr.DIC1 | |
| der. PYC1 | |
| der.ACS1 | |
| GRFura3tTA | *tTA::Leu2, tetO*prom*::sdh*2prom, *tetO*prom*::idh1*prom, |
| repr.SDH2 | *tetO*prom*::dic1*prom, *der.PYC1::pyc1, der.ACS1::acs1,* |
| repr.IDH1 | *der.CIT1::cit1, his3, ura3, leu2* |
| repr.DIC1 | |
| der. PYC 1 | |
| der.ACS 1 | |
| der.CIT1 | |
| GRFura3tTA | *tTA::Leu2, tetO*prom::*sdh*2prom, *tetO*prom*::idh1*prom, |
| repr.SDH2 | *tetO*prom::*dic1*prom, *der.PYC1::pyc1, der.ACS1::acs1,* |
| repr.IDH1 | *der.CIT1::cit1, der.ACO1::aco1, his3, ura3, leu2* |
| repr.DIC1 | |
| der. PYC1 | |
| der.ACS1 | |
| der.CIT1 | |
| der.ACO1 | |
| GRFura3tTA | *tTA::Leu2, tetO*prom*::sdh*2prom, *tet*Oprom*::idh1*prom, |
| repr.SDH2 | *tetO*prom*::dic1*prom*, der.PYC1::pyc1, der.ACS1::acs1,* |
| repr.IDH1 | *der.CIT1::cit1, der.ACO1::aco1, der.ICL1::icl1, his3, ura3, leu2* |
| repr.DIC1 | |
| der. PYC1 | |
| der.ACS1 | |
| der.CIT1 | |
| der.ACO1 | |
| der.ICL1 | |
| GRFura3tTA | *tTA::Leu2, tetOprom::sdh2prom, tetO*prom*::idh1*prom, |
| repr.SDH2 | *tetO*prom::*dic1*prom, *der.PYC1::pyc1, der.ACS1::acs1,* |
| repr.IDH1 | *der.CIT1::cit1, der.ACO1::aco1, der.ICL1::icl1, der.MLS1::mls1,* |
| repr.DIC1 | *his3, ura3, leu2* |
| der. PYC1 | |
| der.ACS1 | |
| der.CIT1 | |
| der.ACO1 | |
| der.ICL1 | |
| der.MLS1 | |
| GRFura3tTA | *tTA::Leu2, tetO*prom*::sdh2*prom, *tetO*prom*::idh1*prom, |
| repr.SDH2 | *tet*Oprom*::dic1*prom, *der.PYC1::pyc1, der.ACS1::acs1,* |
| repr.IDH1 | *der.CIT1::cit1, der.ACO1::aco1, der.ICL1::icl1, der.MLS1::mls1,* |
| repr.DIC1 | *der.MDH3::mdh3, his3, ura3, leu2* |
| der. PYC1 | |
| der.ACS1 | |
| der.CIT1 | |
| der.ACO1 | |
| der.ICL1 | |
| der.MLS1 | |
| der.MDH3 | |
| GRFura3tTA | *tTA::Leu2, tetO*prom*::sdh2*prom, *tetO*prom*::idh1*prom, |
| repr.SDH2 | *tetO*prom*::dic1*prom, *der.PYC1::pyc1, der.ACS1::acs1,* |
| repr.IDH1 | *der.CIT1::cit1, der.ACO1::aco1, der.ICL1::icl1, der.MLS1::mls1,* |
| repr.DIC1 | *der.MDH3::mdh3, der.CIT2::cit2, his3, ura3, leu2* |
| der. PYC1 | |
| der.ACS1 | |
| der.CIT1 | |
| der.ACO1 | |
| der.ICL1 | |
| der.MLS1 | |
| der.MDH3 | |
| der.CIT2 | |

### Beispiel 3: Expression des transkriptionellen Transaktivators unter der Kontrolle des CMV-Promotors am Locus LEU2 in S. cerevisiae GRFura3.

Die kodierende Nukleinsäuresequenz für die tTA-Expressionskassette wurde durch PCR unter Verwendung von Standardmethoden amplifiziert, so dass das resultierende Fragment aus folgenden Komponenten besteht: loxP-kanMX-loxP-CMVprom.-tTA (YEAST 20: 1255-1262, 2003). Als Primer wurden Oligonukleotid-Sequenzen ausgewählt, die an den 5' und 3' Überhängen je die 5' oder die 3' Sequenz des *LEU2-*Gens enthalten und im annealenden Bereich die Sequenzen 5' der loxP-Region und 3' des *tTA-*Gens*.* So ist gewährleistet, dass einerseits das gesamte Fragment inklusive KanR und *tTA* amplifiziert werden und anderseits dieses Fragment anschließend in Hefe transformiert werden kann und durch homologe Rekombination dieses gesamte Fragment in den LEU2-Genlocus der Hefe integriert.

Als Selektionsmarker dient die Resistenz gegen G418. Der resultierende Stamm *S. cerevisiae* GRFura3 *tTA* enthält eine Kopie des Genes *tTA* unter der Kontrolle des CMV-Promotors. Um die Resistenz gegen G418 anschließend wieder zu entfernen, wird der entstandene Hefestamm mit dem cre Rekombinase Vektor pSH47 (Guldener U, Heck S, Fielder T, Beinhauer J, Hegemann JH. (1996) A new efficient gene disruption cassette for repeated use in budding yeast. Nucleic Acids Res. Jul 1;24(13): 2519-24.) transformiert. Durch diesen Vektor wird die cre Rekombinase in der Hefe exprimiert, was zur Folge hat, dass der Sequenz-Bereich innerhalb der beiden loxP-Sequenzen heraus rekombiniert. Dies hat zur Folge, dass lediglich eine der beiden loxP-Sequenzen und die *CMV*prom.-*tTA* Kassete in dem ursprünglichen LEU2-Genlocus enthalten bleibt. Die Folge ist, das der Hefestamm die G418-Resistenz wieder verliert und damit geeignet ist, weitere Gene mittels dieses cre-lox Systems in den Hefestamm zu integrieren bzw. zu entfernen. Der Vektor pSH47 kann daraufhin durch eine Gegenselektion auf YNB-Agarplatten supplimentiert mit Uracil (20 mg/L) und FOA (5-Fluoroorotic acid) (1 g/L) wieder entfernt werden. Dazu müssen die Zellen, die dieses Plasmid tragen, zunächst unter nicht selektiven Bedingungen kultiviert werden und anschließend auf FOA-haltigen Selektivplatten angezogen werden. Unter diesen Bedingungen können lediglich Zellen wachsen, die nicht in der Lage sind, Uracil selbst zu synthetisieren. Dies sind in diesem Fall Zellen, die kein Plasmid (pSH47) mehr enthalten.

### Beispiel 4: Expression von SDH2 unter der Kontrolle des Tetracyclin-regulierbaren tetO₇-Promotors in S. cerevisiae GRFura3.

Die kodierende Nukleinsäuresequenz für die *tetO₇*-Promotorkassette wurde durch PCR unter Verwendung von Standardmethoden amplifiziert, so dass das resultierende Fragment aus folgenden Komponenten besteht: loxP-kanMX-loxP-ADH1term.-tetO₇operator-CYC1prom (YEAST 13:837-848 (1997); Nucleic Acid Research 24(13):1519-2524 (1996)). Als Primer wurden Oligonukleotid-Sequenzen ausgewählt, die an den 5' und 3' Überhängen je die 5' oder die 3' Sequenz des nativen Promotors des *SDH2-*Gens enthalten und im annealenden Bereich die Sequenzen 5' der loxP-Region und 3' des CYClprom. So ist gewährleistet, dass einerseits das gesamte Fragment inklusive KanR und tetO₇-Promotor amplifiziert werden und anderseits dieses Fragment anschließend in Hefe transformiert werden kann und durch homologe Rekombination dieses gesamte Fragment in den *SDH2*-Genlocus der Hefe, vor den codierenden Bereich des Gens *SDH2,* integriert.

Als Selektionsmarker dient die Resistenz gegen G418. Der resultierende Stamm *S. cerevisiae* GRFura3 repr.SDH2 enthält eine Kopie des Genes *SDH2* unter der Kontrolle des Tetracyclin-regulierten tetO₇-Promotor und des nativen *SDH2*-Terminators. Um die Resistenz gegen G418 anschließend wieder zu entfernen, wird der entstandene Hefestamm mit dem cre Rekombinase Vektor pSH47 (Guldener U, Heck S, Fielder T, Beinhauer J, Hegemann JH. (1996) A new efficient gene disruption cassette for repeated use in budding yeast. Nucleic Acids Res. Jul 1;24(13): 2519-24.) transformiert. Durch diesen Vektor wird die cre Rekombinase in der Hefe exprimiert, was zur Folge hat, dass der Sequenz-Bereich innerhalb der beiden loxP-Sequenzen heraus rekombiniert. Dies hat zur Folge, dass lediglich eine der beiden loxP-Sequenzen und die tetO₇-Promotorkassete vor der codierenden Sequenz des Gens *SDH2* enthalten bleibt. Die Folge ist, das der Hefestamm die G418-Resistenz wieder verliert und damit geeignet ist, weitere Gene mittels dieses cre-lox Systems in den Hefestamm zu integrieren bzw. zu entfernen. Der Vektor pSH47 kann daraufhin durch eine Gegenselektion auf YNB-Agarplatten supplimentiert mit Uracil (20 mg/L) und FOA (5-Fluoroorotic acid) (1 g/L) wieder entfernt werden. Dazu müssen die Zellen, die dieses Plasmid tragen, zunächst unter nicht selektiven Bedingungen kultiviert werden und anschließend auf FOA-haltigen Selektivplatten angezogen werden. Unter diesen Bedingungen können lediglich Zellen wachsen, die nicht in der Lage sind, Uracil selbst zu synthetisieren. Dies sind in diesem Fall Zellen, die kein Plasmid (pSH47) mehr enthalten.

### Beispiel 5: Expression von IDH1 unter der Kontrolle des Tetracyclin-regulierbaren tetO₇-Promotors in S. cerevisiae GRFura3.

Die kodierende Nukleinsäuresequenz für die *tetO*₇-Promotorkassette wurde durch PCR unter Verwendung von Standardmethoden amplifiziert, so dass das resultierende Fragment aus folgenden Komponenten besteht: loxP-kanMX-loxP-ADH1term.-tetO₇operator-CYC1prom (YEAST 13:837-848 (997); Nucleic Acid Research 24(13):1519-2524 (1996)). Als Primer wurden Oligonukleotid-Sequenzen ausgewählt, die an den 5' und 3' Überhängen je die 5' oder die 3' Sequenz des nativen Promotors des *IDH1-Gens* enthalten und im annealenden Bereich die Sequenzen 5' der loxP-Region und 3' des CYClprom. So ist gewährleistet, dass einerseits das gesamte Fragment inklusive KanR und tetO₇-Promotor amplifiziert werden und anderseits dieses Fragment anschließend in Hefe transformiert werden kann und durch homologe Rekombination dieses gesamte Fragment in den *IDH1*-Genlocus der Hefe, vor den codierenden Bereich des Gens *IDH1,* integriert.

Als Selektionsmarker dient die Resistenz gegen G418. Der resultierende Stamm *S*. *cerevisiae* GRFura3 repr.IDH1 enthält eine Kopie des Genes *IDH1* unter der Kontrolle des Tetracyclin-regulierten tetO₇-Promotors und des nativen *IDH1-Terminators.* Um die Resistenz gegen G418 anschließend wieder zu entfernen, wird der entstandene Hefestamm mit dem cre Rekombinase Vektor pSH47 (Guldener U, Heck S, Fielder T, Beinhauer J, Hegemann JH. (1996) A new efficient gene disruption cassette for repeated use in budding yeast. Nucleic Acids Res. Jul 1;24(13): 2519-24.) transformiert. Durch diesen Vektor wird die cre Rekombinase in der Hefe exprimiert, was zur Folge hat, dass der Sequenz-Bereich innerhalb der beiden loxP-Sequenzen heraus rekombiniert. Dies hat zur Folge, dass lediglich eine der beiden loxP-Sequenzen und die tetO₇-Promotorkassete vor der codierenden Sequenz des Gens *IDH1* enthalten bleibt. Die Folge ist, das der Hefestamm die G418-Resistenz wieder verliert und damit geeignet ist, weitere Gene mittels dieses cre-lox Systems in den Hefestamm zu integrieren bzw. zu entfernen. Der Vektor pSH47 kann daraufhin durch eine Gegenselektion auf YNB-Agarplatten supplimentiert mit Uracil (20 mg/L) und FOA (5-Fluoroorotic acid) (1 g/L) wieder entfernt werden. Dazu müssen die Zellen, die dieses Plasmid tragen, zunächst unter nicht selektiven Bedingungen kultiviert werden und anschließend auf FOA-haltigen Selektivplatten angezogen werden. Unter diesen Bedingungen können lediglich Zellen wachsen, die nicht in der Lage sind, Uracil selbst zu synthetisieren. Dies sind in diesem Fall Zellen, die kein Plasmid (pSH47) mehr enthalten.

### Beispiel 6: Expression von DIC1 unter der Kontrolle des Tetracyclin-regulierbaren tetO₇-Promotors in S. cerevisiae GRFura3.

Die kodierende Nukleinsäuresequenz für die *tetO*₇-Promotorkassette wurde durch PCR unter Verwendung von Standardmethoden amplifiziert, so dass das resultierende Fragment aus folgenden Komponenten besteht: loxP-kanMX-loxP-ADH1term.-tetO₇operator-CYC1prom (YEAST 13:837-848 (997); Nucleic Acid Research 24(13):1519-2524 (1996)). Als Primer wurden Oligonukleotid-Sequenzen ausgewählt, die an den 5' und 3' Überhängen je die 5' oder die 3' Sequenz des nativen Promotors des *DIC1*-Gens enthalten und im annealenden Bereich die Sequenzen 5' der loxP-Region und 3' des CYC1prom. So ist gewährleistet, dass einerseits das gesamte Fragment inklusive KanR und tetO₇-Promotor amplifiziert werden und anderseits dieses Fragment anschließend in Hefe transformiert werden kann und durch homologe Rekombination dieses gesamte Fragment in den *DIC1*-Genlocus der Hefe, vor den codierenden Bereich des Gens *DIC1,* integriert.

Als Selektionsmarker dient die Resistenz gegen G418. Der resultierende Stamm *S. cerevisiae* GRFura3 repr.DIC1 enthält eine Kopie des Genes *DIC1* unter der Kontrolle des Tetracyclin-regulierten tetO₇-Promotors und des nativen *DIC1*-Terminators. Um die Resistenz gegen G418 anschließend wieder zu entfernen, wird der entstandene Hefestamm mit dem cre Rekombinase Vektor pSH47 (Guldener U, Heck S, Fielder T, Beinhauer J, Hegemann JH. (1996) A new efficient gene disruption cassette for repeated use in budding yeast. Nucleic Acids Res. Jul 1;24(13): 2519-24.) transformiert. Durch diesen Vektor wird die cre Rekombinase in der Hefe exprimiert, was zur Folge hat, dass der Sequenz-Bereich innerhalb der beiden loxP-Sequenzen heraus rekombiniert. Dies hat zur Folge, dass lediglich eine der beiden loxP-Sequenzen und die tetO₇-Promotorkassete vor der codierenden Sequenz des Gens *IDH1* enthalten bleibt. Die Folge ist, das der Hefestamm die G418-Resistenz wieder verliert und damit geeignet ist, weitere Gene mittels dieses cre-lox Systems in den Hefestamm zu integrieren bzw. zu entfernen. Der Vektor pSH47 kann daraufhin durch eine Gegenselektion auf YNB-Agarplatten supplimentiert mit Uracil (20 mg/L) und FOA (5-Fluoroorotic acid) (1 g/L) wieder entfernt werden. Dazu müssen die Zellen, die dieses Plasmid tragen, zunächst unter nicht selektiven Bedingungen kultiviert werden und anschließend auf FOA-haltigen Selektivplatten angezogen werden. Unter diesen Bedingungen können lediglich Zellen wachsen, die nicht in der Lage sind, Uracil selbst zu synthetisieren. Dies sind in diesem Fall Zellen, die kein Plasmid (pSH47) mehr enthalten.

### Beispiel 7: Expression von PYC1, ACS1, CIT1, ACO1, CIT2, ICL1, MLS1 und MDH3 unter der Kontrolle des konstitutiven ADH1-Promotors in S. cerevisiae GRFura3tTArepr.SDH2repr.IDH1repr.DIC1.

Nachfolgende Beschreibung gilt für die sequenzielle Durchführung der Integration von transkripionell deregulierten Genen für die aufgeführten Gene nach gleichem Ablaufschema.

Die kodierenden Nukleinsäuresequenz für die Expressionskassette aus ADH1prom - *PYC1 (ACS1, CIT1, ACO1, CIT2, ICL1, MLS1, MDH3) -* TRP1term. wurde durch PCR unter Verwendung von Standardmethoden aus dem Vektor pFlat-*PYC1 (ACS1, CIT1, ACO1, CIT2, ICL1, MLS1, MDH3)* amplifiziert. Das erhaltene DNA-Fragment wurde nach einer Klenow-Behandlung in den Vekor pUG6 in die EcoRV-Schnittstelle Blunt-end einkloniert und ergab den Vektor pUG6-*PYC1 (ACS1, CIT1, ACO1, CIT2, ICL1, MLS1, MDH3) .* Nach Plasmidisolation wurde ein erweitertes Fragment aus dem Vektor *pUG6-PYC1 (ACS1, CIT1, ACO1, CIT2, ICL1, MLS1, MDH3)* mittels PCR amplifiziert, so dass das resultierende Fragment aus folgenden Komponenten besteht: loxP-kanMX-loxP-ADH1prom- *PYC1 (ACS1, CIT1, ACO1, CIT2, ICL1, MLS1,* MDH3)-Tryptophan-Terminator. Als Primer wurden Oligonukleotid-Sequenzen ausgewählt, die an den 5' und 3' Überhängen je die 5' oder die 3' Sequenz des *PYC1 (ACS1, CIT1, ACO1, CIT2, ICL1, MLS1, MDH3)* -Gens enthalten und im annealenden Bereich die Sequenzen 5' der loxP-Regionen und 3' des Tryptophan-Terminators. So ist gewährleistet, dass einerseits das gesamte Fragment inklusive KanR und *PYC1 (ACS1, CIT1, ACO1, CIT2, ICL1, MLS1, MDH3)* amplifiziert werden und anderseits dieses Fragment anschließend in Hefe transformiert werden kann und durch homologe Rekombination dieses gesamte Fragment entsprechend in den *PYC1 (ACS1, CIT1, ACO1, CIT2, ICL1, MLS1, MDH3*)-Genlocus der Hefe integriert.

Als Selektionsmarker dient jeweils die Resistenz gegen G418. Der final resultierende Stamm *S. cerevisiae* GRFura3tTArepr.SDH2repr.IDH1repr.DIC1dereg.PYC1ACS1CIT1ACO 1ICL1MLS1MDH3CIT2 enthält je eine Kopie der Gene *SDH2, IDH1* und *DIC1* unter der Kontrolle des tetO₇-Promotor und der nativen Terminatoren und je eine Kopie der Gene *PYC1, CIT1, ACS1, ACO1, ICL1, MLS1, MDH3 und CIT2* unter der Kontrolle des konstitutiven ADH1 Promotors und des TRP1 Terminators. Um die Resistenz gegen G418 anschließend jeweils wieder zu entfernen, wird der jeweils entstandene Hefestamm mit dem cre Rekombinase Vektor pSH47 (Guldener U, Heck S, Fielder T, Beinhauer J, Hegemann JH. (1996) A new efficient gene disruption cassette for repeated use in budding yeast. Nucleic Acids Res. Jul 1;24(13): 2519-24.) transformiert. Durch diesen Vektor wird die cre Rekombinase in der Hefe exprimiert, was zur Folge hat, dass der Sequenz-Bereich innerhalb der beiden loxP-Sequenzen heraus rekombiniert. Dies hat zur Folge, dass lediglich eine der beiden loxP-Sequenzen und die jeweilige Expressions-Kassette in dem ursprünglichen entsprechenden Genlocus enthalten bleibt. Die Folge ist, das der Hefestamm die G418-Resistenz wieder verliert und damit geeignet ist, weitere Gene mittels dieses cre-lox Systems in den Hefestamm zu integrieren bzw. zu entfernen. Der Vektor pSH47 kann daraufhin durch eine Gegenselektion auf YNB-Agarplatten supplimentiert mit Uracil (20 mg/L) und FOA (5-Flüoroorotic acid) (1 g/L) wieder entfernt werden. Dazu müssen die Zellen, die dieses Plasmid tragen, zunächst unter nicht selektivien Bedingungen kultiviert werden und anschließend auf FOA-haltigen Selektivplatten angezogen werden. Unter diesen Bedingungen können lediglich Zellen wachsen, die nicht in der Lage sind, Uracil selbst zu synthetisieren. Dies sind in diesem Fall Zellen, die kein Plasmid (pSH47) mehr enthalten.

### Beispiel 8: Bestimmung des Bernsteinsäuregehaltes in Hefekulturen des vorstehend beschriebenen erfindungsgemäßen Hefestammes im Vergleich zum entsprechenden Kontroll-Stamm S. cerevisiae GRFura3

Der vorstehend beschrieben erfindungsgemäße Hefestamm und *S. cerevisiae* GRFura3 werden jeweils für 48 Stunden in WMVIII-Medium (Lang und Lomann, 1995) bei 28°C und 160 rpm in einem 20 ml Kulturvolumen kultiviert. Anschließend werden 500 *µ*l dieser Vorkultur in eine 50 ml Hauptkultur des gleichen Mediums überführt und jeweils für 2 Tage bei 28°C und 160 rpm in einem Schikanekolben kultiviert. Nach Gewinnung des zellfreien Kulturüberstandes werden der Gehalt an Bernsteinsäure, Essigsäure und Ethanol mittels HPCL Analyse bestimmt. Es ergeben sich bei dem erfindungsgemäßen Hefestamm stark erhöhte Bernsteinsäurekonzentrationen, verglichen mit dem Vergleichsstamm.

### Beispiel 9: Mikroorganismus mit konstitutiv aktivem reduktivem Citratzyklus zur Herstellung von organischen Säuren, insbesondere Bernsteinsäure

Der reduktive Citratzyklus, also der nicht cyclische rückwärtigen Umlauf des Citratzyklus ausgehend von Pyruvat über Oxalacetat, Malat und Fumarat zu Succinat (Figur 3 f.) ist Voraussetzung für die maximale molare Ausbeute bei der Produktion von Bernsteinsäure auf Glucose. Nur der reduktive Citratzyklus in Kombination mit dem Glyoxylatzyklus ermöglicht bei der Produktion von Bernsteinsäure maximale Ausbeuten von bis zu 1,71 mol Succinat pro mol Glucose durch Reoxidation von NADH zu NAD+ und CO₂ Fixierung.

Die Umsetzung der transkriptionellen Deregulation des Gens *PYC1* ist unter Beispiel 2 und Beispiel 7 ausgeführt. *PYC1* kodiert für eine der beiden Pyruvat-Carboxylasen, welche die CO2 fixierende Umsetzung von Pyruvat zu Oxalacetat katalysieren, was den ersten Schritt des reduktiven Citratzyklus darstellt.

Den nächsten Schritt, also die Umsetzung von Oxalcetat zu Malat, katalysiert die cytosolische Malat-Dehydrogenase (Mdh2p), welche einem durch Glucose-induzierten verstärkten proteolytischen Abbau unterliegt.

Diese protetolytische Degradation wird verhindert, indem die Malat-Dehydrogenase (Mdh2p) als ein um die ersten 12 N-terminalen Aminosäuren verkürztes Mdh2p Protein exprimiert wird.

Dazu wird ein zu Beginn des "open reading frames" entsprechend verkürztes *MDH2* Gen (*MDH2t,* t= truncated, verkürzt) aus chromosomaler Hefe-DNA des Stammes *S. cerevisiae* S288c mittels PCR amplifiziert und mit Restriktionslinkern versehen und anschließend in das Hefe Chromosom unter der Kontrolle des konstitutiven *ADH1* Promotors integriert. Für die deregulierte Expression dieses verkürzten Gens in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glucose und Ethanol unabhängigen, Expression führt (Lang und Looman, 1995).

Der nächste Schritt bei der Succinat-Produktion über den reduktiven Teil des Citratzyklus ist die Reaktion von Malat zu Fumarat. Katalysiert wird diese Umsetzung durch das Enzym Fumarase (Fumlp). Die Umsetzung von Malat zu Fumarat ist thermodynamisch sehr ungünstig, da die Fumarase eine 17-fach höhere Affinität zu Fumarat, als zu Malat aufweist.

Deshalb wird ein Fumarase-Enzym heterolog in der Hefe Saccharomyces cerevisiae exprimiert, welche aus einem Organismus stammt, welcher natürlicherweise eine gemischte Säuregärung betreibt, da die Thermodymamik der Fumarase solcher Organismen im Bezug auf die Umsetzung von Malat zu Fumarat viel günstiger ist, als in Saccharomyces cerevisiae.

Dazu wird das Gen *fumB* aus chromosomaler bakterieller DNA des Stammes *E. coli* JM109 mittels PCR amplifiziert und mit Restriktionslinkern versehen und anschließend in das Hefe Chromosom unter der Kontrolle des konstitutiven *ADH1* Promotors integriert. Für die deregulierte Expression dieses Gens in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glucose und Ethanol unabhängigen, Expression führt (Lang und Looman, 1995).

Der letzte Schritt zur Succinat-Produktion über den reduktiven Citratzyklus ist die Umsetzung von Fumarat zu Succinat. Diese Reaktion wird im Cytosol durch das Enzym Fumarat-Reduktase (Frdslp) katalysiert. Eine Überexpression dieses Enzyms steigert die Effizienz der reduktiven Succinat-Produktion. Dazu wird das *FRDS1* Gen aus chromosomaler Hefe-DNA des Stammes *S. cerevisiae* S288C mittels PCR amplifiziert und mit Restriktionslinkern versehen und anschließend in das Hefe Chromosom unter der Kontrolle des konstitutiven *ADH1* Promotors integriert. Für die deregulierte Expression dieses verkürzten Gens in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glucose und Ethanol unabhängigen, Expression führt (Lang und Looman, 1995).

Die Glucose-induzierte protetolytische Degradation bzw. Inaktivierung durch Phosphorylierung der Hefe-eigenen Isocitrat-Lyase, einem der Hauptenzyme des Glyoxylatzyklus, wird verhindert, indem eine heterologe Isocitrat-Lyase in der Hefe Saccharomyces cerevisiae exprimiert wird. Dieses Enzym stammt aus einem Mikroorganismus, welcher natürlicherweise einen Glyoxylatzyklus aufweist und "Crabtree" negativ ist, da eine Isocitrat-Lyase aus einem solchen Organismus nicht der durch Glucose-induzierten negativen Regulation, bzw. der proteolytischen Degradation auf Proteinebene unterliegt. Ein aktiver Glyolylatzyklus ist für eine effiziente Produktion von organischen Säuren mit hohen Ausbeuten auf primären Kohlenstoffquellen essentiell.

Dazu wird das Gen *aceA* aus bakterieller DNA des Stammes *E. coli* JM109 mittels PCR amplifiziert und mit Restriktionslinkern versehen und anschließend in das Hefe Chromosom unter der Kontrolle des konstitutiven *ADH1* Promotors integriert. Für die deregulierte Expression dieses Gens in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glucose und Ethanol unabhängigen, Expression führt (Lang und Looman, 1995) .

### Beispiel 10: Herstellung eines Mikroorganismus mit verminderter bzw. inaktivierter Katabolit- bzw. Glucose-Repression zur Herstellung von organischen Säuren des respirativen Zentralmetabolismus

Die folgend beschriebenen Maßnahmen können grundsätzlich zusätzlich zu den Maßnahmen der vorstehend beschriebenen Beispiele angebracht sein, da damit in allen Varianten verbesserte Ausbeuten erhalten werden.

Die verminderte Glucose-Repression in Anwesenheit von Glucose wird durch gezielte Beeinflussung geeigneter Transkriptionsfaktoren und Kinasen, welche für die Glucose-Repression verantwortlich sind, erreicht. Auf diese Weise wird die Effizienz der Produktion organischer Carbonsäuren, insbesondere Dicarbonsäuren und Hydroxyfettsäuren des respirativen Zentralmetabolismus der Hefe, wie zum Beispiel Bernsteinsäure, Fumarsäure, Apfelsäure, Oxalessigsäure hinsichtlich der Produktionsdauer und -ausbeuten auf primären Kohlenstoffquellen, wie z.B. Glucose und anderen Hexose, sowie Pentosen, gesteigert.

Diese Steigerung der Produktionseffizienz organischer Carbonsäuren auf primären Kohlenstoffquellen ergibt sich aus folgenden Eigenschaften, welche ein Stamm mit verminderter oder aufgehobener Glucose-Repression im Vergleich zum Wildtyp aufweist:
▪ der Kohlenstofffluss durch den Citrat- und Glyoxylatzyklus ist erhöht
▪ der Stamm weist ein höheres und schnelleres Wachstum auf
▪ weniger Nebenproduktbildung in Form von Ethanol und Glycerin
▪ der Glyoxylatzyklus wird propagiert
▪ die Enzyme, welche für die CO₂ Fixierung im Zentralmetabolismus verantwortlich sind, werden verstärkt exprimiert
▪ sämtliche Gene des respirativen Systems, z.B. der mitochondrialen Transportsysteme, der Atmungskette, der mitochondrialen Biogenese und für den Austausch von Redoxequivalenten sind aktiv
▪ Enzyme des Citrat- und Glyoxylatzyklus werden der Glucose-induzierten negativen Regulation auf Proteinebene entzogen

Um eine gezielte Verminderung der Glucose-Repression in Anwesenheit von Glucose oder anderen primären Kohlenstoffquellen zu erreichen, werden 2 Gene transkriptionell dereguliert bzw. überexprimiert, welche in Anwesenheit von Glucose stark reprimiert sind. Für die deregulierte Expression dieser Gene in der Hefe *Saccharomyces cerevisiae* wird der konstitutive *ADH1* Promotor verwendet, der durch Modifizierung der natürlichen Sequenz über einen sehr langen Zeitraum zu einer konstitutiven, von Glucose und Ethanol unabhängigen, Expression führt (Lang und Looman, 1995).

Transkriptionell dereguliert werden die Gene *HAP4* und *SAK1.* Hap4p ist Teil des transkriptionellen Aktivatorkomplexes Hap2/3/4/5 und Saklp die Hauptkinase für die Phosphorylierung und somit Aktivierung des Snf1/Snf4-Komplexes. Zu diesem Zweck wurde ein Expressionssystem verwendet, welches die gleichzeitige transkripionell deregulierte Expression von *HAP4* und *SAK1* ermöglicht.

Die zwei Gene (*HAP4* und *SAK1*) werden aus chromosomaler Hefe-DNA des Stammes *S. cerevisiae* S288c mittels PCR amplifiziert und mit Restriktionslinkern versehen und anschließend einzeln in das Hefe Chromosom unter der Kontrolle des konstitutiven *ADH1* Promotors integriert.

Um eine gezielte Verminderung der Glucose-Repression in Anwesenheit von Glucose oder anderen primären Kohlenstoffquellen zu erreichen, wird des Weiteren ein Gen der Glucose-Repressionskaskade deletiert oder durch regulierbare Promotoren kontrolliert. Es handelt sich um das Gen *REG1,* welches die regulatorische Untereinheit der Glc7p/Reglp Typ1 Protein-Phosphatase kodiert. Eine nicht aktive Glc7p/Reglp Phosphatase führt dazu, dass der Snf1/Snf4-Komplex phosphoryliert und somit aktiv bleibt, was erheblich zur Reduzierung bzw. Aufhebung der Glucose Repression beiträgt.

Da ein inaktives Gen *REG1* zu Wachstumsdefekten in der Hefe Saccharomyces cerevisiae führt, wird diesem Gen der durch Tetracyclin-regulierte tetO-Promotor vorgeschaltet. Während der Wachstumsphase des Carbonsäure-Produktionsprozesses wird das Gen *REG1* unter der Kontrolle des durch Tetracyclin reprimierbaren tetO Promotors exprimiert. Während der Produktionsphase wird die Genexpression und somit die Enzymaktivität von Reglp durch Zugabe eines Kultivierungszusatzes, z.B. Tetracyclin, zum Kulturmedium reguliert ausgeschaltet.

*Saccharomyces cerevisiae* wird so verändert, dass der natürliche Promotor des Zielgens durch den reprimierbaren Promotor z.B. durch zielgerichtete Integration ins Hefegenom ausgetauscht wird, und dass der Hefestamm wie unter Beispiel 1 beschrieben den für das tetO-Promotorsystem nötigen transkriptionellen Transaktivator (tTA) enthält.

Die praktische Realisierung eines unter dem tetO-Promotors regulierten Gens REG1 kann ganz entsprechend den Beispielen 4 bis 6 erfolgen, wobei lediglich an Stelle der dort als von dem tetO-Promotor kontrollierten Gene das Gen REG1 einzusetzen ist.

## Patentansprüche

1. Isolierte genetisch veränderte Hefe, wobei das natürliche Gen MDH2 durch eine trunkierte Variante des Gens MDH2 ersetzt oder ergänzt ist,
wobei die trunkierte Variante des Gens MDH2 codiert für ein MDH2 Protein, welches um die ersten 12 N-terminalen Aminosäuren verkürzt ist,
und wobei das natürliche Fumarase Gen durch das Fumarase Gen aus einem von der Hefe verschiedenen Organismus ersetzt oder ergänzt ist, und wobei das Gen FRDS1 überexprimiert wird, und,eines der Gene oder beide Gene PYC1 und PYC2 überexprimiert wird.

2. Isolierte genetisch veränderte Hefe gemäß Anspruch 1, wobei das natürliche Gen MDH2 durch eine trunkierte Variante des Gens MDH2 ersetzt oder ergänzt ist, wobei die trunkierte Variante des Gens MDH2 codiert für ein MDH2 Protein, welches um die ersten 12 N-terminale Aminosäuren verkürzt ist,
und das natürliche Fumarase Gen durch das Fumarase Gen aus einem von der Hefe verschiedenen Organismus ersetzt oder ergänzt ist, und wobei das Gen FRDS1 überexprimiert wird, und wobei das Gen PYC1 überexprimiert wird.

3. Hefe nach Anspruch 1,
wobei die trunkierte Variante des Gens MDH2 codiert für ein MDH2 Protein, bei welchem zusätzlich das C-terminale Prolin verkürzt ist.

4. Hefe nach Anspruch 1,
ausgewählt aus der Gruppe bestehend aus "Saccharomyces cerevisiae, Saccharomyces, Saccharomycecopsis, Saccharomycodes, Schizosaccharomyces, Wickerhamia, Debayomyces, Hansenula, Hanseniaspora, Pichia, Kloeckera, Candida, Zygosaccharomyces, Ogataea, Kuraishia, Komagataella, Yarrowia, Metschnikowia, Williopsis, Nakazawaea, Kluyveromyces, Cryptococcus, Torulaspora, Bullera, Rhodotorula, Willopsis, Kloeckera und Sporobolomyces", ist, und wobei das Gen FUM1 aus einem Organismus aus der Gruppe bestehend aus "Escherichia coli, Anaerobiospirillum succiniciproducens, Actinobacillus succinogenes, Mannheimia succiniciproducens, Corynebacterium glutamicum" ausgewählt ist.

5. Hefe nach Anspruch 1,
wobei zumindest eines der Gene aus der Gruppe bestehend aus "FRDS1, PYC1 und PYC2" unter der Kontrolle eines zweiten fremden, konstitutiv aktiven Promotors, beispielsweise des ADH1-Promotors, steht.

6. Hefe nach Anspruch 6,
wobei beide natürlichen Gene MDH2 und Fumaraseersetzt sind und wobei beide Gene FRDS1 und PYC1 überexprimiert sind.

7. Verwendung einer Hefe nach einem der Ansprüche 1 bis 6 in einem Verfahren zur Herstellung von Bernsteinsäure mit den folgenden Verfahrensstufen:
a) die Hefe wird optional in einem Wachstumskulturmedium unter aeroben Bedingungen kultiviert und vermehrt,
b) anschließend wird die Hefe unter optional anaeroben Bedingungen kultiviert und
c) anschließend an Stufe b) oder während der Stufe b) wird die Bernsteinsäure aus dem Kulturüberstand abgetrennt und optional aufgereinigt.

8. Verwendung einer Hefe nach einem der Ansprüche 1 bis 6 in einem Verfahren zur Herstellung von Bernsteinsäure gemäß Anspruch 7,
wobei die Stufe a) bis zu einer Zelldichte von zumindest 100 g Biotrockenmasse/l, vorzugsweise zumindest 120 g Biotrockenmasse/l, höchstvorzugsweise zumindest 140 g Biotrockenmasse/l, durchgeführt wird.

9. Verwendung einer Hefe nach einem der Ansprüche 1 bis 6 in einem Verfahren zur Herstellung von Bernsteinsäure gemäß Anspruch 7,
wobei die Stufe b) bis zu einer Bernsteinsäurekonzentration von zumindest 0,4 Mol/l, vorzugsweise zumindest 0,8 Mol/l, höchstvorzugsweise zumindest 1,0 Mol/l, durchgeführt wird.

10. Verwendung einer Hefe nach einem der Ansprüche 1 bis 6 in einem Verfahren zur Herstellung von Bernsteinsäure gemäß Anspruch 7,
wobei die Stufe a) bei einer Temperatur von 20 bis 35 °C, vorzugsweise von 28 bis 30 °C, und für eine Dauer von 1 bis 1000 h, vorzugsweise von 2 bis 500 h, höchstvorzugsweise 2 bis 200 h, durchgeführt wird.

11. Verwendung einer Hefe nach einem der Ansprüche 1 bis 6 in einem Verfahren zur Herstellung von Bernsteinsäure gemäß Anspruch 7,
wobei die Stufe b) bei einer Temperatur von 15 bis 40 °C, vorzugsweise von 20 bis 35 °C, und für eine Dauer von 1 bis 1000 h, vorzugsweise von 2 bis 500 h, höchstvorzugsweise 2 bis 200 h, durchgeführt wird.

12. Verfahren zur Herstellung einer Hefe nach Anspruch 5, wobei in der wild-Typ Hefe oder in einer gentechnisch gegenüber dem wild-Typ veränderten oder mutierten Ausgangshefe eines der wild-Typ Gene MDH2 und Fumarase deletiert oder inaktiviert oder ergänzt wird,
wobei die respektive Variante des MDH2 Gens und das fremde Fumarase Gen in die Hefe eingeführt und die Hefe hiermit transformiert wird und
wobei zumindest eines der Gene FRDS1, PYC1 und PYC2, vorzugsweise zumindest beide Gene FRDS1 und PYC1, unter der Kontrolle des zweiten Promotors in die Hefe eingeführt und die Hefe hiermit transformiert wird.

13. Verwendung einer Hefe nach einem der Ansprüche 1 bis 6 zur Herstellung von Bernsteinsäure.

## Claims

1. An isolated genetically modified yeast, wherein the natural gene MDH2 is replaced or supplemented by a truncated variant of the gene MDH2,
wherein the truncated variant of the gene MDH2 encodes an MDH2 protein, which is shortened by the first 12 N-terminal amino acids,
and wherein the natural fumarase gene is replaced or supplemented by the fumarase gene from an organism different from the yeast, and wherein the gene FRDS1 is overexpressed, and one of the genes or both genes PYC1 and/or PYC2 is overexpressed.

2. An isolated genetically modified yeast according to claim 1, wherein the natural gene MDH2 is replaced or supplemented by a truncated variant of the gene MDH2,
wherein the truncated variant of the gene MDH2 encodes an MDH2 protein, which is shortened by the first 12 N-terminal amino acids,
and the natural fumarase gene is replaced or supplemented by the fumarase gene from an organism different from the yeast, and wherein the gene FRDS1 is overexpressed, and wherein the gene PYC1 is overexpressed.

3. The yeast according to claim 1,
wherein the truncated variant of the gene MDH2 encodes an MDH2 protein, in which additionally the C-terminal proline is shortened.

4. The yeast according to claim 1,
selected from the group consisting of "Saccharomyces cerevisiae, Saccharomyces, Saccharomycecopsis, Saccharomycodes, Schizosaccharomyces, Wickerhamia, Debayomyces, Hansenula, Hanseniaspora, Pichia, Kloeckera, Candida, Zygosaccharomyces, Ogataea, Kuraishia, Komagataella, Yarrowia, Metschnikowia, Williopsis, Nakazawaea, Kluyveromyces, Cryptococcus, Torulaspora, Bullera, Rhodotorula, Willopsis, Kloeckera and Sporobolomyces", and wherein the gene FUM1 is selected from an organism from the group consisting of "Escherichia coli, Anaerobiospirillum succiniciproducens, Actinobacillus succinogenes, Mannheimia succiniciproducens, Corynebacterium glutamicum".

5. The yeast according to claim 1,
wherein at least one of the genes from the group consisting of "FRDS1, PYC1 and PYC2" is under the control of a second foreign, constitutively active promoter, for example of the ADH1 promoter.

6. The yeast according to claim 6,
wherein both natural genes MDH2 and FUM1 are replaced, and wherein both genes FRDS1 and PYC1 are overexpressed.

7. Use of a yeast according to one of claims 1 to 6 in a method for producing succinic acid, comprising the following steps:
a) optionally, the yeast is cultured and propagated in a growth culture medium under aerobic conditions,
b) then the microorganism is cultured under optionally anaerobic conditions, and
c) following to step b) or during step b) the succinic acid is separated from the culture supernatant and is optionally purified.

8. Use of a yeast according to one of claims 1 to 6 in a method for producing succinic acid according to claim 7,
wherein step a) is performed up to a cell density of at least 100 g dry biomass/1, preferably at least 120 g dry biomass/1, most preferably at least 140 g dry biomass/1.

9. Use of a yeast according to one of claims 1 to 6 in a method for producing succinic acid according to claim 7,
wherein step b) is performed up to a succinic acid concentration of at least 0.4 mol/l, preferably at least 0.8 mol/l, most preferably at least 1.0 mol/l.

10. Use of a yeast according to one of claims 1 to 6 in a method for producing succinic acid according to claim 7,
wherein step a) is performed at a temperature from 20 to 35 °C, preferably from 28 to 30 °C, and for a period of time from 1 to 1,000 h, preferably from 2 to 500 h, most preferably from 2 to 200 h.

11. Use of a yeast according to one of claims 1 to 6 in a method for producing succinic acid according to claim 7,
wherein step b) is performed at a temperature from 15 to 40 °C, preferably from 20 to 35 °C, and for a period of time from 1 to 1,000 h, preferably from 2 to 500 h, most preferably from 2 to 200 h.

12. A method for producing a yeast according to claim 5, wherein in the wild-type yeast or in an original yeast genetically modified or mutated compared to the wild-type, one of the wild-type genes MDH2 or fumarase is deleted or inactivated or supplemented,
wherein the respective variant of the MDH2 gene and the foreign fumarase gene is introduced into the yeast, and the yeast is transformed therewith, and
wherein at least one of the genes FRDS1, PYC1 and PYC2, preferably at least both genes FRDS1 and PYC1, is introduced under the control of the second promoter into the yeast, and the yeast is transformed therewith.

13. Use of a yeast according to one of claims 1 to 6 for producing succinic acid.

## Revendications

1. Levure génétiquement modifiée isolée, dans laquelle le gène naturel MDH2 est remplacé ou additionné par une variante tronquée du gène MDH2,
dans laquelle la variante tronquée du gène MDH2 code pour une protéine MDH2, qui est raccourcie par les premiers 12 acides aminés N-terminaux,
et dans laquelle le gène naturel fumarase est remplacé ou additionné par le gène fumarase à partir d'un organisme différent de la levure, et dans laquelle le gène FRDS1 est surexprimé, et dans laquelle un des gènes ou les deux gènes PYC1 et/ou PYC2 est surexprimé.

2. Levure génétiquement modifiée isolée selon la revendication 1, dans laquelle le gène naturel MDH2 est remplacé ou additionné par une variante tronquée du gène MDH2, dans laquelle la variante tronquée du gène MDH2 code pour une protéine MDH2, qui est raccourcie par les premiers 12 acides aminés N-terminaux,
et le gène naturel fumarase est remplacé ou additionné par le gène fumarase à partir d'un organisme différent de la levure, et dans laquelle le gène FRDS1 est surexprimé, et dans laquelle le gène PYC1 est surexprimé.

3. Levure selon la revendication 1,
dans laquelle la variante tronquée du gène MDH2 code pour une protéine MDH2, dans laquelle en addition le proline C-terminal est raccourci.

4. Levure selon la revendication 1,
choisie à partir du groupe consistant en "Saccharomyces cerevisiae, Saccharomyces, Saccharomycecopsis, Saccharomycodes, Schizosaccharomyces, Wickerhamia, Debayomyces, Hansenula, Hanseniaspora, Pichia, Kloeckera, Candida, Zygosaccharomyces, Ogataea, Kuraishia, Komagataella, Yarrowia, Metschnikowia, Williopsis, Nakazawaea, Kluyveromyces, Cryptococcus, Torulaspora, Bullera, Rhodotorula, Willopsis, Kloeckera et Sporobolomyces", et dans laquelle le gène FUM1 est choisi à partir d'un organisme du groupe consistant en "Escherichia coli, Anaerobiospirillum succiniciproducens, Actinobacillus succinogenes, Mannheimia succiniciproducens, Corynebacterium glutamicum".

5. Levure selon la revendication 1,
dans laquelle au moins un des gènes à partir du groupe consistant en "FRDS1, PYC1 et PYC2" est sous le contrôle d'un deuxième promoteur étranger constitutivement actif, par exemple du promoteur ADH1.

6. Levure selon la revendication 6,
dans laquelle les deux gènes naturels MDH2 et FUM1 sont remplacés et dans laquelle les deux gènes FRDS1 et PYC1 sont surexprimés.

7. Utilisation d'une levure selon une des revendications 1 à 6 dans un procédé pour produire de l'acide succinique, comprenant les étapes suivantes:
a) optionnellement, la levure est mise en culture et reproduite dans un milieu de culture de croissance sous des conditions aérobies,
b) puis la levure est mise en culture sous optionnellement des conditions anaérobies, et
c) après l'étape b) ou pendant l'étape b) l'acide succinique est séparé du surnagé de la culture et optionnellement est purifié.

8. Utilisation d'une levure selon une des revendications 1 à 6 dans un procédé pour produire de l'acide succinique selon la revendication 7,
dans laquelle l'étape a) est exécutée jusqu'à une densité des cellules d'au moins 100 g biomasse sèche/l, de préférence d'au moins 120 g biomasse sèche/l, de préférence la plus haute d'au moins 140 g biomasse sèche/l.

9. Utilisation d'une levure selon une des revendications 1 à 6 dans un procédé pour produire de l'acide succinique selon la revendication 7,
dans laquelle l'étape b) est exécutée jusqu'à une concentration de l'acide succinique d'au moins 0,4 mole/l, de préférence d'au moins 0,8 mole/l, de préférence la plus haute d'au moins 1,0 mole/l.

10. Utilisation d'une levure selon une des revendications 1 à 6 dans un procédé pour produire de l'acide succinique selon la revendication 7,
dans laquelle l'étape a) est exécutée à une température entre 20 et 35 °C, de préférence entre 28 et 30 °C, et pendant un temps entre 1 et 1.000 h, de préférence entre 2 et 500 h, de préférence la plus haute entre 2 et 200 h.

11. Utilisation d'une levure selon une des revendications 1 à 6 dans un procédé pour produire de l'acide succinique selon la revendication 7,
dans laquelle l'étape b) est exécutée à une température entre 15 et 40 °C, de préférence entre 20 et 35 °C, et pendant un temps entre 1 et 1.000 h, de préférence entre 2 et 500 h, de préférence la plus haute entre 2 et 200 h.

12. Procédé pour produire une levure selon la revendication 5, dans laquelle dans la levure du type sauvage ou dans une levure initiale génétiquement modifiée ou mutée en comparaison avec le type sauvage, un des gènes du type sauvage MDH2 ou fumarase est supprimé ou inactivé ou additionné,
dans laquelle la variante respective du gène MDH2 et/ou le gène étranger fumarase est introduit dans la levure, et la levure est donc transformée, et
dans laquelle au moins un des gènes FRDS1, PYC1 et PYC2, de préférence au moins les deux gènes FRDS1 et PYC1, est introduit sous le contrôle du deuxième promoteur dans la levure, et la levure est donc transformée.

13. Utilisation d'une levure selon une des revendications 1 à 6 pour produire de l'acide succinique.
